# EUROPEAN PATENT SPECIFICATION

(11) **EP 2 776 553 B1**
(45) Date of publication and mention of the grant of the patent: **11.07.2018**
(21) Application number: 12847544.9
(22) Date of filing: 07.11.2012
(51) Int. Cl.: C12M 1/34

(54) **BIOMARKERS FOR SANFILIPPO SYNDROME AND USES THEREOF**
BIOMARKER FÜR DAS SANFILIPPO-SYNDROM UND ANWENDUNGEN DAVON
BIOMARQUEURS POUR LE SYNDROME DE SANFILIPPO ET LEURS UTILISATIONS

(30) Priority: 07.11.2011 US 201161556810 P; 06.01.2012 US 201261584165 P
(43) Date of publication of application: 17.09.2014
(73) Proprietor: Shire Human Genetic Therapies, Inc., Lexington MA 02421 (US)
(72) Inventor: HASLETT, Patrick, Somerville, MA 02143 (US); RICHARD, Charlie, Carlisle, MA 01741 (US)
(74) Representative: Carpmaels & Ransford LLP
(86) International application number: PCT/US2012/063935
(87) International publication number: WO 2013/070760

(56) References cited:
- WO-A1-2007/065273
- WO-A2-2010/078515
- US-A1- 2005 048 047
- US-A1- 2005 055 733
- US-A1- 2009 092 996
- US-A1- 2009 233 354
- US-A1- 2010 028 370
- US-A1- 2010 184 013
- US-A1- 2011 008 810
- NESTRASIL IGOR ET AL: "Brain volumes and cognitive function in MPS IIIA: Cross-sectional study", MOLECULAR GENETICS AND METABOLISM, vol. 102, no. 2, February 2011 (2011-02), page S32, XP002738254, & 7TH ANNUAL WORLD SYMPOSIUM OF THE LYSOSOMAL-DISEASE-NETWORK; LAS VEGAS, NV, USA; FEBRUARY 16 -18, 2011 ISSN: 1096-7192
- VERÓNICA DELGADILLO ET AL: "Genistein supplementation in patients affected by Sanfilippo disease", JOURNAL OF INHERITED METABOLIC DISEASE, KLUWER ACADEMIC PUBLISHERS, DO, vol. 34, no. 5, 10 May 2011 (2011-05-10), pages 1039-1044, XP019952748, ISSN: 1573-2665, DOI: 10.1007/S10545-011-9342-4
- EWA PIOTROWSKA ET AL: "Two-year follow-up of Sanfilippo Disease patients treated with a genistein-rich isoflavone extract: Assessment of effects on cognitive functions and general status of patients", MEDICAL SCIENCE MONITOR, vol. 17, no. 4, 1 April 2011 (2011-04-01), pages CR196-CR202, XP55084478, ISSN: 1234-1010, DOI: 10.12659/MSM.881715

## Description

### BACKGROUND

Mucopolysaccharidosis III (MPS-III), also known as Sanfilippo syndrome, is a rare autosomal recessive lysosomal storage disease, caused by a deficiency in one of the enzymes needed to break down the glycosaminoglycan (formerly called mucopolysaccharide), heparan sulfate. Heparan sulfate is an essential cell surface glycoprotein and a critical component in forming and maintaining the extra-cellular matrix, and with multiple roles in cell surface receptor-ligand interactions. Four different types of MPS-III have been identified: MPS-III A, B, C and D (also known as Sanfilippo syndrome Type A, B, C and D, respectively). The four MPS-III types are each distinguished by a different enzyme deficiency. Syndrome Type A (MPS-III A) has been shown to occur as a result of over 70 different mutations so far discovered in the heparan N-sulfatase gene, which reduce or abolish enzyme function. Syndrome Type B (MSP-III B) has been linked to a disruption in the enzyme N-acetyl-alpha-D-glucosaminidase. Syndrome Type C (MPS-III C) has been linked to acetyl-CoA:alpha-glucosaminide acetyltransferease. Syndrome Type D (MPS-III D) has been linked to the enzyme N-acetylglucosamine-G-sulfate sulfatase. All of these enzymes are involved in heparan sulfate regulation.

The incidence of Sanfilippo syndrome widely varies geographically, with an average incidence rate of approximately 1 in 100,000 births, with Syndrome Type A (MPS-III A) constituting 60% of all cases. Due to the nature of the genetic disease it manifests during early childhood development, and results in a life-span that does not usually extend beyond late teens to early twenties. Despite the different genetic causes for the disease, diagnosis and staging for the syndrome is problematic, since all four types of MPS-III are considered to be clinically indistinguishable. Infants appear normal, and children may later develop some mild facial dysmorphism. Stiff joints and coarse hair typically associated with other forms of mucopolysaccharidosis are usually not present until later on in the disease. After a relatively symptom-free infancy and very early childhood, patients usually present with a slowing of development and/or behavioral problems, followed by progressive intellectual decline resulting in severe dementia, progressive loss of motor skills and ultimately a loss in mobility. The subtle and non-specific onset of clinical features, with predominantly neurological symptoms, make the illness difficult to diagnose in the early stages. The current methods used to diagnose MSP-III are assays for measuring enzyme levels and gene sequencing, both of which are expensive and time consuming. In addition, primary accumulation of heparan sulfate triggers poorly understood pathological cascade with primary CNS manifestations, which makes it difficult to accurately assess the severity of the disease using existing diagnostic methods and provide optimal treatment for Sanfilippo syndrome patient.

Therefore, there is a need in the field to develop more effective biomarkers as indicators for Sanfilippo syndrome and the severity of the syndrome.

### SUMMARY

The present disclosure provides biomarkers for efficient, accurate and patient friendly characterization of Sanfilippo syndrome. As discussed in the examples below, the present invention is, in part, based on the discovery of various biomarkers associated with Sanfilippo syndrome. Without wishing to be bound by any particular theory, it is contemplated that these biomarkers may illustrate pathological cascade and/or mechanisms underlying the Sanfilippo syndrome development and progression. Thus, these biomarkers, used alone or in combination, may permit more accurate robust characterization of Sanfilippo syndrome, resulting in more precise determination of the types and/or severity of the syndrome. In addition, biomarkers can be used to effectively monitor treatment response in Sanfilippo syndrome patients and/or to optimize treatment for Sanfilippo syndrome.

In one aspect, the present disclosure provides a method for characterizing Sanfilippo syndrome, which comprises measuring a level of one or more biomarkers in a sample obtained from a subject, wherein the one or more biomarkers are selected from those listed in Table 1 and combination thereof, and determining the severity of Sanfilippo syndrome in the subject, based on the measured level of the one or more biomarkers as compared to a control level. In some embodiments, the one or more biomarkers comprise at least two, three, four, five, six, seven, eight, nine, ten, eleven, twelve, thirteen, fourteen, fifteen, sixteen, seventeen, eighteen, or nineteen biomarkers selected from Table 1.

In some embodiments, the one or more biomarkers comprise at least one biomarker selected from Tau, phospho-Tau (p181), Calbindin D-28K, or Hepatocyte Growth Factor. In some embodiments, the one or more biomarkers comprise at least two biomarkers selected from Tau, phospho-Tau (p181), Calbindin D-28K, or Hepatocyte Growth Factor. In some embodiments, the one or more biomarkers comprise at least three biomarkers selected from Tau, phospho-Tau (p181), Calbindin D-28K, or Hepatocyte Growth Factor. In some embodiments, the one or more biomarkers comprise Tau, phospho -Tau (p181), and Hepatocyte Growth Factor. In some embodiments, the one or more biomarkers comprise Tau, phospho-Tau (p181), Calbindin D-28K, and Hepatocyte Growth Factor.

In some embodiments, the protein expression level of the one or more biomarkers is measured. In some embodiments, the proteins expression level is measured by performing an immuno assay using one or more antibodies that specifically bind the one or more biomarkers. In some embodiments, the protein expression level of the one or more biomarkers is measured by performing 2D-gel electrophroresis. In some embodiments, the nucleic acid expression level of the one or more biomarkers is measured. In some embodiments the nucleic acid expression level of the one or more biomarkers is measure by hybridization. In some embodiments, the nucleic acid expression level of the one or more biomarkers is measured by amplification. In further embodiments, the amplification method for measuring the nucleic acid expression level of the one or more biomarkers is RT-PCR amplification. In yet further embodiments, the method used for measuring the nucleic acid expression level of the one or more biomarkers is selected from the group consisting of, Nucleic Acid sequence based amplification (NASBA), Transcription Medicated Amplification (TMA), Quantitative PCR (qPCR), Real-time PCR, Loop-Mediated Isothermal Amplification (LAMP), TaqMan, Invader, InvaderPlus, Rolling Circle, Strand Displacement Amplification (SDA), Q-Beta-Replicase, Helicase Dependent Amplification (HDA), Branched DNA, Hydrolysis FRET probes, Ligase Chain Reaction (LCR), degenerate oilgonucleotide primed PCR, or other methods known to those of skill in the art.

In some embodiments, the sample used for measuring the one or more biomarkers is a biological sample. In some embodiments, the sample is obtained from cerebrospinal fluid (CSF), cells, tissue, whole blood, mouthwash, plasma, serum, urine, stool, saliva, cord blood, chronic villus sample culture, amniotic fluid, amniotic fluid culture, transcervical lavage fluid, and combinations thereof. In some embodiments, the sample is from the group consisting of, cerebrospinal fluid (CSF), cells, tissue, whole blood, mouthwash, plasma, serum, urine, stool, saliva, cord blood, chronic villus sample culture, amniotic fluid, amniotic fluid culture, or transcervical lavage fluid. In some embodiments, the sample is a cerebrospinal fluid (CSF) sample. In some embodiments, the sample is a peripheral blood sample.

In some embodiments, the one or more biomarkers are compared to a control level, wherein the control level is indicative of the level of one or more biomarkers in a control subject who does not have Sanfilippo syndrome. In some embodiments, the control level is the level of the one or more biomarkers measured under conditions in a control sample obtained from one or more healthy control subjects. In some embodiments, the control sample is a pooled sample from a plurality of healthy control subjects. In yet further embodiments, the control level is a numerical reference based on historical data.

In some embodiments, the one or more biomarkers in the sample are elevated as compared to the control level. In some embodiments, the difference between the levels of the one or more biomarkers measured in the sample and the control level, correlates with the severity of Sanfilippo syndrome in the subject. In some embodiments, the control level is indicative of the level of the one or more biomarkers in a control subject who is suffering from Sanfilippo syndrome with a known severity. In some embodiments, the control level is a numerical threshold established based on historical data to indicate a pre-determined Sanfilippo syndrome disease stage. In some embodiments, the control level is indicative or a pre-determined type of Sanfilippo syndrome.

In some embodiments, the one or more biomarkers are used in conjunction with one or more additional markers. In some embodiments, the one or more additional markers are selected from the group consisting of Glycosaminoglycan (GAG) (e.g. - Heparan Sulfate), beta-hexosaminidase, Lysosomal-associated membrane protein 1, Lysosomal-associated membrane protein 2 and combinations thereof. In some embodiments, the step of determining the severity of Sanfilippo syndrome comprises determining if the Sanfilipo syndrome in the subject is type A, B, C or D. In some embodiments, the method for diagnosising Sanfilippo syndrome in a subject, further comprises a step of identifying a treatment regimen for the subject.

In another aspect, the present disclosure provides a method for monitoring treatment response in a Sanfilippo syndrome patient comprising, measuring a level of one or more biomarkers in a sample obtained from a Sanfilippo syndrome patient after receiving treatment for Sanfilippo syndrome, wherein the one or more biomarkers are selected from those listed in Table 1, Glycosaminoglycan (GAG), heparan sulfate, and combinations thereof, and maintaining or adjusting the treatment based on the measured level of the one or more biomarkers as compared to a control level. In some embodiments, the step of maintaining or adjusting the treatment comprises maintaining doses and/or frequencies of the treatment. In some embodiments, the step of maintaining or adjusting the treatment comprises increasing doses and/or frequencies of the treatment. In some embodiments, the step of maintaining or adjusting the treatment comprises reducing doses and/or frequencies of the treatment.

In some embodiments, the control level is the level of the one or more biomarkers in the Sanfilippo syndrome patient before receiving the treatment. In some embodiments, the control level is the level of the one or more biomarkers in the Sanfilippo syndrome patient measured at an earlier time point during the treatment. In some embodiments, the control level is the level of the one or more biomarkers in a control patient without the treatment. In some embodiments, level of the one or more biomarkers in the Sanfilippo syndrome patient is diminished, as compared to the control patient. In some embodiments, the diminished level of the one or more biomarkers indicates that the patient has a positive response to the treatment. In some embodiments, the Sanfilippo syndrome is selected from the group consisting of Type A, B, C or D. In particular embodiments, the Sanfilippo syndrome is Sanfilippo syndrome Type A.

In some embodiments, the treatment is enzyme replacement therapy. In some embodiments, the enzyme replacement therapy comprises administering a recombinant heparan N-sufatase (HNS) protein. In other embodiments, the enzyme replacement therapy comprises administering a recombinant enzyme from the group consisting of Heparan G-sulfatase, N-acetly-alpha-D-glucosaminidase, Acetyl-CoA:alpha-glucosaminide acetyltransferase, N-acetylglucosamine-G-sulfate sulfatase and/or combinations thereof. In some embodiments, the recombinant enzyme is administered by intrathecal delivery. In yet other embodiments, the recombinant HNS protein is administered by intrathecal delivery.

In some embodiments, the sample obtained from a Sanfilippo syndrome patient is selected from the group consisting of cerebrospinal fluid (CSF), cells, tissue, whole blood, plasma, serum, blood, and combinations thereof. In some embodiments, the sample obtained from a Sanfilippo syndrome patient is cerebrospinal fluid (CSF). In some embodiments, the sample obtained from a Sanfilippo syndrome patient is peripheral blood sample.

In some embodiments, the one or more biomarkers comprises Glycosaminoglycan (GAG) heparan sulfate. In some embodiments, the one or more biomarkers comprise at least one biomarker selected from Table 1. In some embodiments, the one or more biomarkers comprise at least one biomarker selected from Tau, phospho-Tau (p181), Calbindin D-28K, or Hepatocyte Growth Factor. In some embodiments, the one or more biomarkers comprise at least two biomarkers selected from Tau, phospho-Tau (p181), Calbindin D-28K, or Hepatocyte Growth Factor. In some embodiments, the one or more biomarkers comprise at least three biomarkers selected from Tau, phospho-Tau (p181), Calbindin D-28K, or Hepatocyte Growth Factor. In some embodiments, the one or more biomarkers comprise Tau, phospho-Tau (p181), and Hepatocyte Growth Factor. In some embodiments, the one or more biomarkers comprise Tau, phospho-Tau (p181), Calbindin D-28K, and Hepatocyte Growth Factor.

In some embodiments, the protein expression level of the one or more biomarkers is measured. In some embodiments, the protein expression level is measure by performing an immuno assay using one or more antibodies that specifically bind to the one or more biomarkers. In some embodiments, the protein expression level of the one or more biomarkers is measured by performing 2D-gel electrophoresis. In some embodiments, the nucleic acid expression level of the one or more biomarkers is measured. In some embodiments the nucleic acid expression level of the one or more biomarkers is measure by hybridization. In some embodiments, the nucleic acid expression level of the one or more biomarkers is measured by amplification. In further embodiments, the amplification method for measuring the nucleic acid expression level of the one or more biomarkers is RT-PCR amplification. In yet further embodiments, the method used for measuring the nucleic acid expression level of the one or more biomarkers is selected from the group consisting of, Nucleic Acid sequence based amplification (NASBA), Transcription Medicated Amplification (TMA), Quantitative PCR (qPCR), Real-time PCR, Loop-Mediated Isothermal Amplification (LAMP), TaqMan, Invader, InvaderPlus, Rolling Circle, Strand Displacement Amplification (SDA), Q-Beta-Replicase, Helicase Dependent Amplification (HDA), Branched DNA, Hydrolysis FRET probes, Ligase Chain Reaction (LCR), degenerate oilgonucleotide primed PCR, or other method known to those of skill in the art.

In another aspect, the present disclosure provides a kit comprising one or more reagents for measuring a level of one or more biomarkers selected from those listed in Table 1 and combinations thereof, and a control level or a control sample for determining the control level of the one or more biomarkers, indicative of a predetermined Sanfilippo syndrome type or stage. In some embodiments, the kit further comprises, an additional control level, or an additional control sample for determining the additional control level, indicative of absence of Sanfilippo syndrome. In some embodiments, the kit further comprises a regent for measuring Glycosaminoglycan (GAG). In some embodiments, the one or more reagents measure the protein expression level of the one or more biomarkers. In some embodiments, the one or more reagents comprise one or more antibodies that specifically bind to the one or more biomarkers. In some embodiments, the one or more reagents measure the nucleic acid level of one or more biomarkers. In further embodiments, the one or more reagents comprise one or more oligonucleotide probes that specifically hybridize to the mRNA of the one or more biomarkers.

In still another aspect, the present disclosure provides a method for monitoring treatment response in a Sanfilippo syndrome patient, comprising: measuring a level of one or more neurodevelopmental biomarkers obtained from a Sanfilippo syndrome patient after receiving treatment for Sanfilippo syndrome, and maintaining or adjusting the treatment based on the measured change in the one or more neurodevelopmental biomarkers as compared to a control subject.

In some embodiments, the one or more neurodevelopmental biomarkers comprise a brain anatomical marker. In some embodiments, the brain anatomical marker is indicative of brain volume. In some embodiments, the brain volume is selected from the group consisting of white matter volume, grey matter volume, cortical volume, compound ventricular + CSF volume, cerebella volume, total brain volume and combination thereof.

In some embodiments, the control level is the level of the one or more brain anatomical markers in the Sanfilippo syndrome patient before receiving the treatment. In some embodiments, the control level is the level of the one or more brain anatomical markers in the Sanfilippo syndrome patient measured at an earlier time point during the treatment. In some embodiments, the control level is the level of the one or more brain anatomical markers in a control patient without the treatment.

In some embodiments, a diminished change of the one or more brain anatomical markers indicates that the patient has positive response to the treatment.

In some embodiments, the Sanfilippo syndrome is Sanfilippo syndrome Type A.

In some embodiments, the treatment is enzyme replacement therapy. In some embodiments, the enzyme replacement therapy comprises administering a recombinant heparan N-sulfatase (HNS) protein. In some embodiments, the recombinant HNS protein is administered by intrathecal delivery.

In some embodiments, the step of maintaining or adjusting treatment comprises maintaining doses and/or frequencies of the treatment. In some embodiments, the step of maintaining or adjusting treatment comprises increasing doses and/or frequencies of the treatment. In some embodiments, the step of maintaining or adjusting treatment comprises reducing doses and/or frequencies of the treatment.

As used in this application, the terms "about" and "approximately" are used as equivalents. Any numerals used in this application with or without about/approximately are meant to cover any normal fluctuations appreciated by one of ordinary skill in the relevant art.

### BRIEF DESCRIPTION OF THE DRAWINGS

The drawings are for illustration purposes only, and not for limitation.
*Figure 1**. Mental Age Equivalence against Calendar Age.* Mental age equivalence was plotted against actual calendar age for subjects with Sanfilippo Syndrome.
*Figure 2**. Age Related Decline in Developmental Quotient.* Exemplary data demonstrating baseline developmental quotient plotted against age (months) to examine age-related decline in subjects with Sanfilippo Syndrome.
*Figure 3**. Age Related Decline in Developmental Quotient.* Additional exemplary data demonstrating baseline developmental quotient plotted against age (years) to examine age-related decline in subjects with Sanfilippo Syndrome.
*Figure 4**. Non-contrast Brian MRI.* Automated volumetric analysis using FreeSurfer software.
*Figure 5**. Change in Cerebral Cortical Volume.* Exemplary data demonstrating change in cerebral cortical volume (grey matter volume) plotted against age (months) in children who were diagnosed with Sanfilippo syndrome before and after age 6.
*Figure 6**. Change in Cerebral Cortical Volume.* Additional exemplary data demonstrating change in cerebral cortical volume (grey matter volume) plotted against age (years) in children who were diagnosed with Sanfilippo syndrome before and after age 6.
*Figure 7**. Change Ventricular and CSF Volume.* Change in ventricular and CSF volume was examined in children who were diagnosed with Sanfilippo syndrome before and after age 6.
*Figure 8**. Change in White Matter.* Change in cerebral cortical volume (white matter volume) was examined in children who were diagnosed with Sanfilippo syndrome before and after age 6.
*Figure 9**. Change in Cerebellar Volume.* Change in cerebella volume was examined in children who were diagnosed with Sanfilippo syndrome before and after age 6.
*Figure 10**. Change in Total Brain Volume.* Change in total brain volume was examined in children who were diagnosed with Sanfilippo syndrome before and after age 6.
*Figure 11**. Cerebral Cortical Volume Vs. Developmental Quotient.* Cerebral cortical volume (grey matter volume) was compared with the developmental quotient for each subject.
*Figure 12**. Ventricular and CSF Volume Vs. Developmental Quotient.* Cerebral cortical volume (grey matter volume) was compared with the developmental quotient for each subject.
*Figure 13**. White Matter Volume Vs. Developmental Quotient.* White matter volume was compared with the developmental quotient for each subject.
*Figure 14**. Total Brain Volume Vs. Developmental Quotient.* Total brain volume was compared with the developmental quotient for each subject.
*Figure 15**. Testing for Heparan Sulfate Glycosaminoglycan using the Shire GAG Assay.* The levels of heparan sulfate glycosaminoclygan in cerebrospinal fluid was determined for each subject in the study using the Shire GAG assay.
*Figure 16**. Comparison of Hepatocyte Growth Factor protein levels.* Samples of cerebrospinal fluid (CSF) was collected from subjects patients with a confirmed diagnosis of MPS-III A (SAN A) and young adult healthy controls (CTRL). CSF was drawn from the lumbar intrathecal space and analyzed using a luminex-based multiplexed bead-based immunoassay. The concentration of Hepatocyte Growth Factor protein within each sample was determined.
*Figure 17**. Comparison of Calbindin D protein levels.* Samples of cerebrospinal fluid (CSF) was collected from subjects patients with a confirmed diagnosis of MPS-III A (SAN A) and young adult healthy controls (CTRL). CSF was drawn from the lumbar intrathecal space and analyzed using a luminex-based multiplexed bead-based immunoassay. The concentration of Calbindin D protein within each sample was determined.
*Figure 18**. Comparison of total Tau protein levels.* Samples of cerebrospinal fluid (CSF) was collected from subjects patients with a confirmed diagnosis of MPS-III A (SAN A) and young adult healthy controls (CTRL). CSF was drawn from the lumbar intrathecal space and analyzed using a luminex-based multiplexed bead-based immunoassay. The concentration of total Tau protein within each sample was determined.
*Figure 19**. Comparison of phosphorylated Tau protein levels.* Samples of cerebrospinal fluid (CSF) was collected from subjects patients with a confirmed diagnosis of MPS-III A (SAN A) and young adult healthy controls (CTRL). CSF was drawn from the lumbar intrathecal space and analyzed using a luminex-based multiplexed bead-based immunoassay. The concentration of phosphorylated Tau protein within each sample was determined.

### DEFINITIONS

In order for the present invention to be more readily understood, certain terms are first defined. Additional definitions for the following terms and other terms are set forth throughout the specification.

*Antibody:* As used herein, the term "antibody" refers to a polypeptide consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light chains are typically classified as either kappa or lambda. Heavy chains are typically classified as gamma, mu, alpha, delta, or epsilon, which in turn define the immunoglobulin classes, IgG, IgM, IgA, IgD and IgE, respectively. A typical immunoglobulin (antibody) structural unit is known to comprise a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms "variable light chain" (VL) and "variable heavy chain" (VH) refer to these light and heavy chains respectively. An antibody can be specific for a particular antigen. The antibody or its antigen can be either an analyte or a binding partner. Antibodies exist as intact immunoglobulins or as a number of well-characterized fragments produced by digestion with various peptidases. Thus, for example, pepsin digests an antibody below the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region thereby converting the (Fab')2 dimer into an Fab' monomer. The Fab' monomer is essentially an Fab with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of ordinary skill in the art will appreciate that such Fab' fragments may be synthesized de novo either chemically or by utilizing recombinant DNA methodology. Thus, the term "antibody," as used herein also includes antibody fragments either produced by the modification of whole antibodies or synthesized *de novo* using recombinant DNA methodologies. In some embodiments, antibodies are single chain antibodies, such as single chain Fv (scFv) antibodies in which a variable heavy and a variable light chain are joined together (directly or through a peptide linker) to form a continuous polypeptide. A single chain Fv ("scFv") polypeptide is a covalently linked VH::VL heterodimer which may be expressed from a nucleic acid including VH- and VL-encoding sequences either joined directly or joined by a peptide-encoding linker. (See, *e.g.*, Huston, et al. (1988) Proc. Nat. Acad. Sci. USA, 85:5879-5883.) A number of structures exist for converting the naturally aggregated, but chemically separated light and heavy polypeptide chains from an antibody V region into an scFv molecule which will fold into a three dimensional structure substantially similar to the structure of an antigen-binding site. See, e.g. U.S. Pat. Nos. 5,091,513 and 5,132,405 and 4,956,778.

*Array*: The terms *"array", "micro-array*", and *"biochip* " are used herein interchangeably. They refer to an arrangement, on a substrate surface, of hybridizable array elements, preferably, multiple nucleic acid molecules of known sequences. Each nucleic acid molecule is immobilized to a discrete spot (*i.e.*, a defined location or assigned position) on the substrate surface. The term "*micro-array*" more specifically refers to an array that is miniaturized so as to require microscopic examination for visual evaluation.

*Binding agent:* As used herein, the term "binding agent" includes any naturally occurring, synthetic or genetically engineered agent, such as protein, that binds an antigen or a target protein or peptide. Binding agents can be derived from naturally occurring antibodies or synthetically engineered. A binding protein or agent can function similarly to an antibody by binding to a specific antigen to form a complex and elicit a biological response (e.g., agonize or antagonize a particular biological activity). Binding agents or proteins can include isolated fragments, "Fv" fragments consisting of the variable regions of the heavy and light chains of an antibody, recombinant single chain polypeptide molecules in which light and heavy chain variable regions are connected by a peptide linker ("ScFv proteins"), and minimal recognition units consisting of the amino acid residues that mimic the hypervariable region. The term Binding Agent as used herein can also include antibody fragments either produced by the modification of whole antibodies or synthesized *de novo* using recombinant DNA methodologies. In some embodiments, antibodies are single chain antibodies, such as single chain Fv (scFv) antibodies in which a variable heavy and a variable light chain are joined together (directly or through a peptide linker) to form a continuous polypeptide. A single chain Fv ("scFv") polypeptide is a covalently linked VH::VL heterodimer which may be expressed from a nucleic acid including VH- and VL-encoding sequences either joined directly or joined by a peptide-encoding linker. (See, *e.g.*, Huston, et al. (1988) Proc. Nat. Acad. Sci. USA, 85:5879-5883.) A number of structures exist for converting the naturally aggregated, but chemically separated light and heavy polypeptide chains from an antibody V region into an scFv molecule which will fold into a three dimensional structure substantially similar to the structure of an antigen-binding site. See, e.g. U.S. Pat. Nos. 5,091,513 and 5,132,405 and 4,956,778. In some embodiments, the term Binding Agent as used herein can also include antibody. See the definition of Antibody.

*Biomarker.* As defined herein, the term "biomarker" refers to a substance (e.g., protein or nucleic acid) that can be used as an indicator of a disease, risk of developing the disease, carrier status, or responses to a therapeutic intervention. Typically, a suitable biomarker has a characteristic that can be objectively measured and evaluated as an indicator. In some embodiments, a biomarker is an organic biomolecule which is differentially present in a sample taken from a subject of one phenotypic status (e.g., having a disease) as compared with another phenotypic status (e.g., not having the disease). A biomarker is differentially present between different phenotypic statuses if the mean or median expression level of the biomarker in the different groups is calculated to be statistically significant. Common tests for statistical significance include, among others, t-test, ANOVA, Kruskal-Wallis, Wilcoxon, Mann- Whitney, odds ratio, Linear Discriminant Analysis, Quadratic Discriminant Analysis and K-nearest neighbor. Biomarkers, alone or in combination, provide measures of relative risk that a subject belongs to one phenotypic status or another. Therefore, they are useful as markers for disease (diagnostics), therapeutic effectiveness of a drug (theranostics) and drug toxicity.

*Cerebroanatomical Marker:* The term "Cerebroanatomical Marker" as used herein refers to any anatomical feature of a brain. In some embodiments, a cerebroanatomical marker comprises, but is not limited to, any portion of the central nervous system that is enclosed within the cranium, continuous with the spinal cord and composed of gray matter and white matter.

*Compound and Agent:* The terms *"compound"* and *"agent"* are used herein interchangeably. They refer to any naturally occurring or non-naturally occurring (*i.e.*, synthetic or recombinant) molecule, such as a biological macromolecule (*e.g*., nucleic acid, polypeptide or protein), organic or inorganic molecule, or an extract made from biological materials such as bacteria, plants, fungi, or animal (particularly mammalian, including human) cells or tissues. The compound may be a single molecule or a mixture or complex of at least two molecules.

*Control:* As used herein, the term "control" has its art-understood meaning of being a standard against which results are compared. Typically, controls are used to augment integrity in experiments by isolating variables in order to make a conclusion about such variables. In some embodiments, a control is a reaction or assay that is performed simultaneously with a test reaction or assay to provide a comparator. In one experiment, the "test" (*i.e.*, the variable being tested) is applied. In the second experiment, the "control," the variable being tested is not applied. In some embodiments, a control is a historical control (*i.e.*, of a test or assay performed previously, or an amount or result that is previously known). In some embodiments, a control is or comprises a printed or otherwise saved record. A control may be a positive control or a negative control.

*Diagnosis:* As used herein, the term *"diagnosis"* refers to a process aimed at determining if an individual is afflicted with a disease or ailment. In the context of the present invention, *"diagnosis of Sanfilippo syndrome"* refers to a process aimed at one or more of: determining if an individual is afflicted with Sanfilippo syndrome, identifying a Sanfilippo syndrome subtype (*i.e.*, subtype A, B, C or D), and determining the stage of the disease (*e.g.*, early Sanfillipo syndrome or late Sanfillipo syndrome).

*Differentially expressed biomarker:* As used herein, the term "*differentially expressed biomarker,*" when used in connection with Sanfilippo syndrome, refers to a biomarker whose level of expression is different in a subject (or a population of subjects) afflicted with Sanfilippo syndrome relative to its level of expression in a healthy or normal subject (or a population of healthy or normal subjects). The term also encompasses a biomarker whose level of expression is different for a different disease subtype (*i.e.*, Sanfilippo syndrome A,B,C or D). The term further encompasses a biomarker whose level of expression is different at different stages of the disease (*e.g*., mild or early Sanfilippo syndrome, severe or late Sanfilippo syndrome). Differential expression includes quantitative, as well as qualitative, differences in the temporal or cellular expression pattern of the biomarker. As described in greater details below, a differentially expressed biomarker, alone or in combination with other differentially expressed biomarkers, is useful in a variety of different applications in diagnostic, staging, therapeutic, drug development and related areas. The expression patterns of the differentially expressed biomarkers disclosed herein can be described as a fingerprint or a signature of Sanfilippo syndrome, Sanfilippo syndrome subtype, Sanfilippo syndrome stage and Sanfilippo syndrome severity and/or progression. They can be used as a point of reference to compare and characterize unknown samples and samples for which further information is sought. The term *"decreased level of expression",* as used herein, refers to a decrease in expression of at least 10% or more, for example, 20%, 30%, 40%, or 50%, 60%, 70%, 80%, 90% or more, or a decrease in expression of greater than 1-fold, 2-fold, 3-fold, 4-fold, 5-fold, 10-fold, 50-fold, 100-fold or more as measured by one or more methods described herein. The term *"increased level of expression*", as used herein, refers to an increase in expression of at least 10% or more, for example, 20%, 30%, 40%, or 50%, 60%, 70%, 80%, 90% or more or an increase in expression of greater than 1-fold, 2-fold, 3-fold, 4-fold, 5-fold, 10-fold, 50-fold, 100-fold or more as measured by one or more methods, such as method described herein.

*Enzyme replacement therapy (ERT):* As used herein, the term "enzyme replacement therapy (ERT)" refers to any therapeutic strategy that corrects an enzyme deficiency by providing the missing enzyme. In some embodiments, the missing enzyme is provided by intrathecal administration. In some embodiments, the missing enzyme is provided by infusing into bloodsteam. Once administered, enzyme is taken up by cells and transported to the lysosome, where the enzyme acts to eliminate material that has accumulated in the lysosomes due to the enzyme deficiency. Typically, for lysosomal enzyme replacement therapy to be effective, the therapeutic enzyme is delivered to lysosomes in the appropriate cells in target tissues where the storage defect is manifest.

*Effective amount:* As used herein, the term *"effective amount"* refers to an amount of a compound or agent that is sufficient to fulfill its intended purpose(s). In the context of the present invention, the purpose(s) may be, for example: to modulate the expression of at least one inventive biomarker; and/or to delay or prevent the onset of Sanfilippo syndrome; and/or to slow down or stop the progression, aggravation, or deterioration of the symptoms of Sanfilippo syndrome; and/or to alleviate one or more symptoms associated with Sanfilippo syndrome; and/or to bring about amelioration of the symptoms of Sanfilippo syndrome, and/or to cure Sanfilippo syndrome.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control individual (or multiple control individuals) in the absence of the treatment described herein. A "control individual" is an individual afflicted with the same form of lysosomal storage disease (e.g., Sanfilippo syndrome) as the individual being treated, who is about the same age as the individual being treated (to ensure that the stages of the disease in the treated individual and the control individual(s) are comparable).

*Intrathecal administration*: As used herein, the term "intrathecal administration" or "intrathecal injection" refers to an injection into the spinal canal (intrathecal space surrounding the spinal cord). Various techniques may be used including, without limitation, lateral cerebroventricular injection through a burrhole or cisternal or lumbar puncture or the like. In some embodiments, "intrathecal administration" or "intrathecal delivery" according to the present invention refers to IT administration or delivery via the lumbar area or region, i.e., lumbar IT administration or delivery. As used herein, the term "lumbar region" or "lumbar area" refers to the area between the third and fourth lumbar (lower back) vertebrae and, more inclusively, the L2-S1 region of the spine.

*Hybridizing:* The term *"hybridizing"* refers to the binding of two single stranded nucleic acids *via* complementary base pairing. The term *"specific hybridization"* refers to a process in which a nucleic acid molecule preferentially binds, duplexes, or hybridizes to a particular nucleic acid sequence under stringent conditions (*e.g*., in the presence of competitor nucleic acids with a lower degree of complementarity to the hybridizing strand). In certain embodiments of the present invention, these terms more specifically refer to a process in which a nucleic acid fragment (or segment) from a test sample preferentially binds to a particular probe and to a lesser extent or not at all, to other probes, for example, when these probes are immobilized on an array.

*Kit:* As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to a delivery systems comprising two or more separate containers that each contain a subportion of the total kit components. The containers may be delivered to the intended recipient together or separately. for example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contain a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

*Normal:* As used herein, the term "normal," when used to modify the term "individual" or "subject" they refer to an individual or group of individuals who does not have a particular disease or condition and is also not a carrier of the disease or condition. The term "normal" is also used herein to qualify a biological specimen or sample isolated from a normal or wild-type individual or subject, for example, a "normal biological sample."

*Nucleic Acid:* As used herein refers to an oligonucleotide, nucleotide or polynucleotide, and fragments or portions thereof, and to DNA or RNA of genomic or synthetic origin that may be single or double stranded, and represent the sense or antisense strand.

*Nucleic Acid Molecule:* The terms *"nucleic acid molecule"* and *"polynucleotide"* are used herein interchangeably. They refer to a deoxyribonucleotide or ribonucleotide polymer in either single- or double-stranded form, and unless otherwise stated, encompass known analogs of natural nucleotides that can function in a similar manner as naturally occurring nucleotides. The terms encompass nucleic acid-like structures with synthetic backbones, as well as amplification products.

*Protein*: In general, a "protein" is a polypeptide (*i.e.*, a string of at least two amino acids linked to one another by peptide bonds). Proteins may include moieties other than amino acids (*e.g*., may be glycoproteins) and/or may be otherwise processed or modified. Those of ordinary skill in the art will appreciate that a "protein" can be a complete polypeptide chain as produced by a cell (with or without a signal sequence), or can be a functional portion thereof. Those of ordinary skill will further appreciate that a protein can sometimes include more than one polypeptide chain, for example linked by one or more disulfide bonds or associated by other means.

*Probe:* The term "*probe*", as used herein, refers to a nucleic acid molecule of known sequence, which can be a short DNA sequence (*i.e.*, an oligonucleotide), a PCR product, or mRNA isolate. Probes are specific DNA sequences to which nucleic acid fragments from a test sample are hybridized. Probes specifically bind to nucleic acids of complementary or substantially complementary sequence through one or more types of chemical bonds, usually through hydrogen bond formation.

*A reagent that specifically detects expression levels:* As used herein, the term "*a reagent that specifically detects expression levels"* refers to one or more reagents used to detect the expression level of one or more biomarkers. Examples of suitable reagents include, but are not limited to, antibodies capable of specifically binding to a marker protein of interest, nucleic acid probes capable of specifically hybridizing to a polynucleotide sequence of interest, or PCR primers capable of specifically amplifying a polynucleotide sequence of interest. The term "*amplify*" is used herein in the broad sense to mean creating/generating an amplification product. "*Amplification*", as used herein, generally refers to the process of producing multiple copies of a desired sequence, particularly those of a sample.

*Sample*: As used herein, the term "Sample" encompasses any sample obtained from a biological source. The terms "biological sample" and "sample" are used interchangeably. A biological sample can, by way of non-limiting example, include cerebrospinal fluid (CSF), blood, amniotic fluid, sera, urine, feces, epidermal sample, skin sample, cheek swab, sperm, amniotic fluid, cultured cells, bone marrow sample and/or chorionic villi. Convenient biological samples may be obtained by, for example, scraping cells from the surface of the buccal cavity. Cell cultures of any biological samples can also be used as biological samples, e.g., cultures of chorionic villus samples and/or amniotic fluid cultures such as amniocyte cultures. A biological sample can also be, e.g., a sample obtained from any organ or tissue (including a biopsy or autopsy specimen), can comprise cells (whether primary cells or cultured cells), medium conditioned by any cell, tissue or organ, tissue culture. In some embodiments, biological samples suitable for the invention are samples which have been processed to release or otherwise make available a nucleic acid for detection as described herein. Suitable biological samples may be obtained from a stage of life such as a fetus, young adult, adult (e.g., pregnant women), and the like. Fixed or frozen tissues also may be used.

*Subject*: As used herein, the term "subject" refers to a human or any non-human animal (e.g., mouse, rat, rabbit, dog, cat, cattle, swine, sheep, horse or primate). A human includes pre and post natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*System:* The term *"system"* and *"biological system"* are used herein interchangeably. A system may be any biological entity that can express or comprise at least one inventive biomarker. In the context of the present invention, *in vitro*, *in vivo*, and *ex vivo* systems are considered; and the system may be a cell, a biological fluid, a biological tissue, or an animal. For example, a system may originate from a living subject (*e.g*., it may be obtained by drawing blood, or by performing needle biopsy), or from a deceased subject (*e.g*., it may be obtained at autopsy).

*Suffering from*: An individual who is "*suffering from*" a disease, disorder, and/or condition has been diagnosed with or displays one or more symptoms of the disease, disorder, and/or condition.

*Treatment:* As used herein, the term *"treatment"* (also "treat" or "treating") refers to any administration of a therapeutic protein (e.g., lysosomal enzyme) that partially or completely alleviates, ameliorates, relieves, inhibits, delays onset of, reduces severity of and/or reduces incidence of one or more symptoms or features of a particular disease, disorder, and/or condition (e.g., Sanfilippo syndrome). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition.

### DETAILED DESCRIPTION

The present disclosure provides, among other things, biomarkers that differentially expressed in Sanfilippo syndrome patients and methods of using such biomarkers for diagnosis, treatment monitoring and/or optimization. In some embodiments, inventive biomarkers provided herein can be used to diagnose Sanfilippo syndrome (e.g., types A, B, C and D), determine the severity, stage and/or type of the syndrome. In some embodiments, inventive biomarkers provided herein can be used for evaluation of the efficacy of a treatment, monitoring treatment response in a Sanfilippo syndrome patient and/or treatment optimization. Inventive biomarkers provided herein can be used alone or in combination, with or without other biomarkers known to be associated with lysosomal storage diseases, as part of a panel of biomarkers.

Various aspects of the invention are described in detail in the following sections. In this application, the use of "or" means "and/or" unless stated otherwise.

### Biomarkers for Sanfilippo Syndrome

Suitable biomarkers may include any substances (e.g., proteins or nucleic acids) that can be used as an indicator of a disease state of Sanfilippo syndrome, the severity of the syndrome, or responses to a therapeutic intervention. Typically, a suitable biomarker has a characteristic that can be objectively measured and evaluated as an indicator. Typically, a suitable biomarker for Sanfilippo syndrome is differentially expressed between Sanfilippo syndrome patients and normal healthy individuals. Thus, in some embodiments, "differential expression profiling" may be used to identify biomarkers for Sanfilippo syndrome. As used herein, the term "differential expression profiling" refers to methods of comparing the gene or protein expression levels or patterns in two or more samples (e.g., samples obtained from Sanfilippo syndrome patients vs. control samples obtained from healthy control individuals). Typically, a gene or protein is differentially expressed if the difference (e.g., increase or decrease) in the expression level or pattern between two samples is statistically significant (*i.e.*, the difference is not caused by random variations). In some embodiments, a gene or protein is differentially expressed if the difference in the expression level between two samples is more than 20%, 30%, 40%, 50%, 60%, 70%, 80%, 90%, 1-fold, 1.2-fold, 1.5-fold, 1.75-fold, 2-fold, 2.25-fold, 2.5-fold, 2.75-fold, or 3-fold.

As discussed in the Examples section, differential expression profiling was successfully used to identify biomarkers for Sanfilippo syndrome. In particular, it has been discovered that elevated or diminished expression of certain proteins or genes is associated with the presence of Sanfilippo syndrome. Accordingly, analysis of expression and/or activity level of such differentially expressed proteins or genes can be employed to evaluate risk for Sanfilippo syndrome, detect the presence of Sanfilippo syndrome, monitor progression or abatement of Sanfilippo syndrome, and/or monitor treatment response or optimization.

Exemplary biomarkers for Sanfilippo syndrome are listed in Table 1. The protein and nucleic acid (e.g., gene, mRNA and/or cDNA) sequences for the biomarkers listed in Table 1 are known in the art. For example, the protein and nucleic acid sequences for each biomarker listed in Table 1 are available in GenBank and can be readily found by search using the name of each biomarker listed in Table 1.

Biomarkers provided herein can be used alone or in combination as an indicator to evaluate risk for Sanfilippo syndrome, detect the presence of Sanfilippo syndrome, monitor progression or abatement of Sanfilippo syndrome, and/or monitor treatment response or optimization. In some embodiments, individual biomarker described herein may be used. In some embodiments, at least two, three, four, five, six, seven, eight, nine, ten, eleven, twelve, thirteen, fourteen, fifteen, sixteen, seventeen, eighteen, or nineteen biomarkers selected from Table 1 may be used in combination as a panel.

In particular embodiments, suitable biomarkers are selected from Tau, phospho-Tau (p181), Calbindin D-28K, and/or Hepatocyte Growth Factor. For example, any one, two, three biomarkers selected from Tau, phospho-Tau (p181), Calbindin D-28K, or Hepatocyte Growth Factor may be used. In some embodiments, Tau, phospho-Tau (p181), and Hepatocyte Growth Factor may be used. In some embodiments, all four of them (Tau, phospho-Tau (p181), Calbindin D-28K, and Hepatocyte Growth Factor) may be used in combination.

In some embodiments, inventive biomarkers described herein may be used in conjunction with one or more additional markers, in particular, those markers known to be associated with lysosomal storage diseases, in particular, with Sanfilippo syndrome (e.g., Sanfilippo syndrome A, B, C or D). Thus, in some embodiments, one or more biomarkers described herein (e.g., those provided in Table 1) may be used in conjunction with additional markers, such as, for example, glycosaminoglycan (GAG) heparan sulfate, beta-hexosaminidase, LAMP1, LAMP2, to name but a few.

**Table 1. - Exemplary Biomarkers Identified**

| **Biomarker** | **Abbreviation** | **Linear Analysis** | **Quadrati c Analysis** | **Nearest-Neighbor** |
|---|---|---|---|---|
| Alpha-1-Antitrypsin | AAT | - | - | 0.0750 |
| Alpha-2-Macroglobulin | Alpha-2-M | 0.0667 | 0.0000 | 0.0500 |
| Apolipoprotein B | Apo B | - | - | 0.1000 |
| Calbindin | | 0.1000 | 0.0333 | 0.0500 |
| Complement C3 | C3 | - | 0.0583 | 0.0583 |
| Fatty Acid-Binding Protein, heart | H-FABP | - | 0.0583 | 0.0333 |
| Heparin-Binding EGF-Like Growth Factor | HB-EGF | 0.1000 | - | - |
| Hepatocyte Growth Factor | HGF | - | 0.0417 | 0.0167 |
| Kallikrein-7 | KLK-7 | - | 0.0500 | 0.1000 |
| Lysosomal-Associated Membrane Protein 2 | LAMP2 | 0.1000 | 0.1000 | 0.0750 |
| Macrophage Colony-Stimulating Factor 1 | M-CSF | - | 0.1000 | 0.0667 |
| Monocyte Chemotactic Protein 1 | MCP-1 | - | 0.0750 | 0.0500 |
| Sex Hormone-Binding Globulin | SHBG | 0.0667 | 0.0250 | 0.0000 |
| Tau | | - | 0.0333 | 0.0667 |
| Thyroxine-Binding Globulin | TBG | - | 0.0917 | 0.0667 |
| Tumor Necrosis Factor Receptor-Like 2 | TNFR2 | 0.0500 | 0.0833 | 0.0333 |
| Vascular Endothelial Growth Factor Receptor 1 | VEGFR-1 | - | 0.0750 | 0.0583 |
| Vitronectin | | - | - | 0.0500 |
| pTau(181) | | - | 0.0917 | 0.0667 |

### Measuring biomarker levels in a biological sample

A variety of methods may be used to measure biomarker levels in a biological sample. Typically, any characteristic indicative of expression or activity levels for biomarkers may be used. In some embodiments, the protein expression level of a biomarker in a sample is measured. In some embodiments, the nucleic acid expression level of a biomarker in a sample is measured.

### Biological Samples

Methods of the disclosure may be applied to any type of biological samples allowing one or more inventive biomarkers to be assayed. Examples of suitable biological samples include, but are not limited to, cerebrospinal fluid (CSF), cells, tissue, whole blood, mouthwash, plasma, serum, urine, stool, saliva, cord blood, chorionic villus sample, chorionic villus sample culture, amniotic fluid, amniotic fluid culture, transcervical lavage fluid. Biological samples suitable for the inventive may be fresh or frozen samples collected from a subject, or archival samples with known diagnosis, treatment and/or outcome history. Biological samples may be collected by any invasive or non-invasive means, such as, for example, by drawing CSF or blood from a subject, or using fine needle aspiration or needle biopsy, or by surgical biopsy.

In certain embodiments, biological samples may be used without or with limited processing of the sample. For example, protein extract may be prepared from a biological sample. In some embodiments, a protein extract contains the total protein content. In some embodiments, protein extracts containing one or more of membrane proteins, nuclear proteins, and cytosolic proteins may be prepared. Methods of protein extraction are well known in the art (see, for example "Protein Methods", D.M. Bollag et al., 2nd Ed., 1996, Wiley-Liss; "Protein Purification Methods: A Practical Approach", E.L. Harris and S. Angal (Eds.), 1989; "Protein Purification Techniques: A Practical Approach", S. Roe, 2nd Ed., 2001, Oxford University Press; "Principles and Reactions of Protein Extraction, Purification, and Characterization", H. Ahmed, 2005, CRC Press: Boca Raton, FL). Numerous different and versatile kits can be used to extract proteins from bodily fluids and tissues, and are commercially available from, for example, BioRad Laboratories (Hercules, CA), BD Biosciences Clontech (Mountain View, CA), Chemicon International, Inc. (Temecula, CA), Calbiochem (San Diego, CA), Pierce Biotechnology (Rockford, IL), and Invitrogen Corp. (Carlsbad, CA). User Guides that describe in great detail the protocol to be followed are usually included in all these kits. Sensitivity, processing time and costs may be different from one kit to another. One of ordinary skill in the art can easily select the kit(s) most appropriate for a particular situation. After the protein extract has been obtained, the protein concentration of the extract is preferably standardized to a value being the same as that of the control sample in order to allow signals of the protein markers to be quantitated. Such standardization can be made using photometric or spectrometric methods or gel electrophoresis.

In some embodiments, nucleic acids may be extracted from a biological sample. For example, RNA may be extracted from the sample before analysis. Methods of RNA extraction are well known in the art (see, for example, J. Sambrook et al., "Molecular Cloning: A Laboratory Manual", 1989, 2nd Ed., Cold Spring Harbor Laboratory Press: Cold Spring Harbor, NY). Most methods of RNA isolation from bodily fluids or tissues are based on the disruption of the tissue in the presence of protein denaturants to quickly and effectively inactivate RNases. Isolated total RNA may then be further purified from the protein contaminants and concentrated by selective ethanol precipitations, phenol/chloroform extractions followed by isopropanol precipitation or cesium chloride, lithium chloride or cesium trifluoroacetate gradient centrifugations. Kits are also available to extract RNA (*i.e.*, total RNA or mRNA) from bodily fluids or tissues and are commercially available from, for example, Ambion, Inc. (Austin, TX), Amersham Biosciences (Piscataway, NJ), BD Biosciences Clontech (Palo Alto, CA), BioRad Laboratories (Hercules, CA), GIBCO BRL (Gaithersburg, MD), and Qiagen, Inc. (Valencia, CA).

In certain embodiments, after extraction, mRNA is amplified, and transcribed into cDNA, which can then serve as template for multiple rounds of transcription by the appropriate RNA polymerase. Amplification methods are well known in the art (see, for example, A.R. Kimmel and S.L. Berger, Methods Enzymol. 1987, 152: 307-316; J. Sambrook et al., "Molecular Cloning: A Laboratory Manual", 1989, 2nd Ed., Cold Spring Harbour Laboratory Press: New York; "Short Protocols in Molecular Biology", F.M. Ausubel (Ed.), 2002, 5th Ed., John Wiley & Sons; U.S. Pat. Nos. 4,683,195; 4,683,202 and 4,800,159). Reverse transcription reactions may be carried out using non-specific primers, such as an anchored oligo-dT primer, or random sequence primers, or using a target-specific primer complementary to the RNA for each probe being monitored, or using thermostable DNA polymerases (such as avian myeloblastosis virus reverse transcriptase or Moloney murine leukemia virus reverse transcriptase).

### Measuring Protein Expression Levels

Measuring or determination of protein expression levels in a biological sample may be performed by any suitable method (see, for example, E. Harlow and A. Lane, "Antibodies: A Laboratories Manual", 1988, Cold Spring Harbor Laboratory: Cold Spring Harbor, NY).

In general, protein expression levels are determined by contacting a biological sample obtained from a subject with binding agents for one or more of protein biomarkers; detecting, in the sample, the levels of one or more protein biomarkers that bind to the binding agents; and comparing the levels of one or more protein biomarkers in the sample with the levels of the corresponding protein biomarkers in a control sample. As used herein, the term "*binding agent*" refers to an entity such as a polypeptide or antibody that specifically binds to an inventive protein biomarker. An entity "*specifically binds*" to a polypeptide if it reacts/interacts at a detectable level with the polypeptide but does not react/interact detectably with peptides containing unrelated sequences or sequences of different polypeptides.

In certain embodiments, a suitable binding agent is a ribosome, with or without a peptide component, an RNA molecule, or a polypeptide (*e.g.*, a polypeptide that comprises a polypeptide sequence of a protein marker, a peptide variant thereof, or a non-peptide mimetic of such a sequence).

In other embodiments, a suitable binding agent is an antibody specific for a protein biomarker described herein (*e.g.*, an antibody specific for any biomarkers listed in Table 1). In some embodiments, a suitable antibody can specifically bind to a particular form of a protein biomarker, for example, a phosphorated protein (e.g., phospho-Tau (p181)). Suitable antibodies for use in the methods of the present disclosure include monoclonal and polyclonal antibodies, immunologically active fragments (*e.g*., Fab or (Fab)₂ fragments), antibody heavy chains, humanized antibodies, antibody light chains, and chimeric antibodies. Antibodies, including monoclonal and polyclonal antibodies, fragments and chimeras, may be prepared using methods known in the art (see, for example, R.G. Mage and E. Lamoyi, in "Monoclonal Antibody Production Techniques and Applications", 1987, Marcel Dekker, Inc.: New York, pp. 79-97; G. Kohler and C. Milstein, Nature, 1975, 256: 495-497; D. Kozbor et al., J. Immunol. Methods, 1985, 81: 31-42; and R.J. Cote et al., Proc. Natl. Acad. Sci. 1983, 80: 2026-203; R.A. Lerner, Nature, 1982, 299: 593-596; A.C. Nairn et al., Nature, 1982, 299: 734-736; A.J. Czernik et al., Methods Enzymol. 1991, 201: 264-283; A.J. Czernik et al., Neuromethods: Regulatory Protein Modification: Techniques & Protocols, 1997, 30: 219-250; A.J. Czernik et al., Neuroprotocols, 1995, 6: 56-61; H. Zhang et al., J. Biol. Chem. 2002, 277: 39379-39387; S.L. Morrison et al., Proc. Natl. Acad. Sci., 1984, 81: 6851-6855; M.S. Neuberger et al., Nature, 1984, 312: 604-608; S. Takeda et al., Nature, 1985, 314: 452-454). Antibodies to be used in the methods of the disclosure can be purified by methods well known in the art (see, for example, S.A. Minden, "Monoclonal Antibody Purification", 1996, IBC Biomedical Library Series: Southbridge, MA). For example, antibodies can be affinity-purified by passage over a column to which a protein marker or fragment thereof is bound. The bound antibodies can then be eluted from the column using a buffer with a high salt concentration.

Instead of being prepared, antibodies to be used in the methods of the present disclosure may be obtained from scientific or commercial sources (*e.g.*, Cayman Chemical).

*Labeled Binding Agents.* In certain embodiments, the binding agent is directly or indirectly labeled with a detectable moiety. The role of a detectable agent is to facilitate the detection step of the diagnostic method by allowing visualization of the complex formed by binding of the binding agent to the protein marker (or analog or fragment thereof). Preferably, the detectable agent is selected such that it generates a signal which can be measured and whose intensity is related (preferably proportional) to the amount of protein marker present in the sample being analyzed. Methods for labeling biological molecules such as polypeptides and antibodies are well-known in the art (see, for example, "Affinity Techniques. Enzyme Purification: Part B", Methods in Enzymol., 1974, Vol. 34, W.B. Jakoby and M. Wilneck (Eds.), Academic Press: New York, NY; and M. Wilchek and E.A. Bayer, Anal. Biochem., 1988, 171: 1-32).

Any of a wide variety of detectable agents can be used in the practice of the present disclosure. Suitable detectable agents include, but are not limited to: various ligands, radionuclides, fluorescent dyes, chemiluminescent agents, microparticles (such as, for example, quantum dots, nanocrystals, phosphors and the like), enzymes (such as, for example, those used in an ELISA, *i.e.*, horseradish peroxidase, beta-galactosidase, luciferase, alkaline phosphatase), colorimetric labels, magnetic labels, and biotin, dioxigenin or other haptens and proteins for which antisera or monoclonal antibodies are available.

In certain embodiments, the binding agents (*e.g*., antibodies) may be immobilized on a carrier or support (*e.g.*, a bead, a magnetic particle, a latex particle, a microtiter plate well, a cuvette, or other reaction vessel). Examples of suitable carrier or support materials include agarose, cellulose, nitrocellulose, dextran, Sephadex, Sepharose, liposomes, carboxymethyl cellulose, polyacrylamides, polystyrene, gabbros, filter paper, magnetite, ion-exchange resin, plastic film, plastic tube, glass, polyamine-methyl vinyl-ether-maleic acid copolymer, amino acid copolymer, ethylene-maleic acid copolymer, nylon, silk, and the like. Binding agents may be indirectly immobilized using second binding agents specific for the first binding agents (*e.g*., mouse antibodies specific for the protein markers may be immobilized using sheep antimouse IgG Fc fragment specific antibody coated on the carrier or support).

Protein expression levels in a biological sample may be determined using immunoassays. Examples of such assays are time resolved fluorescence immunoassays (TR-FIA), radioimmunoassays, enzyme immunoassays (*e.g*., ELISA), immunofluorescence immunoprecipitation, latex agglutination, hemagglutination, Western blot, and histochemical tests, which are conventional methods well-known in the art. As will be appreciated by one skilled in the art, the immunoassay may be competitive or non-competitive. Methods of detection and quantification of the signal generated by the complex formed by binding of the binding agent with the protein marker will depend on the nature of the assay and of the detectable moiety (*e.g*., fluorescent moiety).

Alternatively, the protein expression levels may be determined using mass spectrometry based methods or image (including use of labeled ligand) based methods known in the art for the detection of proteins. Other suitable methods include 2D-gel electrophoresis, proteomics-based methods. Proteomics, which studies the global changes of protein expression in a sample, can include the following steps: (1) separation of individual proteins in a sample by electrophoresis (1-D PAGE), (2) identification of individual proteins recovered from the gel (*e.g.*, by mass spectrometry or N-terminal sequencing), and (3) analysis of the data using bioinformatics.

### Measuring Nucleic Acid Expression Levels

Measuring or determination of expression levels of nucleic acids in a biological sample may be performed by any suitable method, including, but not limited to, hybridization (e.g., Southern or Northern analysis), polymerase chain reaction (PCR) (see, for example, U.S. Pat Nos., 4,683,195; 4,683,202, and 6,040,166; "PCR Protocols: A Guide to Methods and Applications", Innis et al. (Eds.), 1990, Academic Press: New York), reverse transcriptase PCR (RT-PCT), anchored PCR, competitive PCR (see, for example, U.S. Pat. No. 5,747,251), rapid amplification of cDNA ends (RACE) (see, for example, "Gene Cloning and Analysis: Current Innovations, 1997, pp. 99-115); ligase chain reaction (LCR) (see, for example, EP 01 320 308), one-sided PCR (Ohara et al., Proc. Natl. Acad. Sci., 1989, 86: 5673-5677), *in situ* hybridization, Taqman-based assays (Holland et al., Proc. Natl. Acad. Sci., 1991, 88: 7276-7280), differential display (see, for example, Liang et al., Nucl. Acid. Res., 1993, 21: 3269-3275) and other RNA fingerprinting techniques, nucleic acid sequence based amplification (NASBA) and other transcription based amplification systems (see, for example, U.S. Pat. Nos. 5,409,818 and 5,554,527), Qbeta Replicase, Strand Displacement Amplification (SDA), Repair Chain Reaction (RCR), nuclease protection assays, subtraction-based methods, Rapid-Scan™, and the like.

Nucleic acid probes for use in the detection of polynucleotide sequences in biological samples may be constructed using conventional methods known in the art. Suitable probes may be based on nucleic acid sequences encoding at least 5 sequential amino acids from regions of nucleic acids encoding a biomarker, and preferably comprise about 15 to about 50 nucleotides. A nucleic acid probe may be labeled with a detectable moiety, as mentioned above in the case of binding agents. The association between the nucleic acid probe and detectable moiety can be covalent or non-covalent. Detectable moieties can be attached directly to nucleic acid probes or indirectly through a linker (E.S. Mansfield et al., Mol. Cell. Probes, 1995, 9: 145-156). Methods for labeling nucleic acid molecules are well-known in the art (for a review of labeling protocols, label detection techniques and recent developments in the field, see, for example, L.J. Kricka, Ann. Clin. Biochem. 2002, 39: 114-129; R.P. van Gijlswijk et al., Expert Rev. Mol. Diagn. 2001, 1: 81-91; and S. Joos et al., J. Biotechnol. 1994, 35: 135-153).

Nucleic acid probes may be used in hybridization techniques to detect polynucleotides encoding biomarkers. The technique generally involves contacting and incubating nucleic acid molecules in a biological sample obtained from a subject with the nucleic acid probes under conditions such that specific hybridization takes place between the nucleic acid probes and the complementary sequences in the nucleic acid molecules. Typically, stringent hybridization conditions are used. In some embodiments, "stringent hybridization conditions" refer to hybridization conditions at least as stringent as the following: hybridization in 50% formamide, 5XSSC, 50 mM NaH₂PO₄, pH 6.8, 0.5% SDS, 0.1 mg/mL sonicated salmon sperm DNA, and 5XDenhart's solution at 42 °C overnight; washing with 2XSSC, 0.1% SDS at 45 °C; and washing with 0.2XSSC, 0.1% SDS at 45 °C. In some embodiments, stringent hybridization conditions should not allow for hybridization of two nucleic acids which differ over a stretch of 20 contiguous nucleotides by more than two bases. After incubation, the non-hybridized nucleic acids are removed, and the presence and amount of nucleic acids that have hybridized to the probes are detected and quantified.

Detection of nucleic acid molecules comprising polynucleotide sequences coding for a biomarker may involve amplification of specific polynucleotide sequences using an amplification method such as PCR (e.g., RT-PCR), followed by analysis of the amplified molecules using techniques known in the art. Suitable primers can be routinely designed by one skilled in the art. In order to maximize hybridization under assay conditions, primers and probes employed in the methods of the disclosure generally have at least 60%, preferably at least 75% and more preferably at least 90% identity to a portion of nucleic acids encoding a biomarker.

Hybridization and amplification techniques described herein may be used to assay qualitative and quantitative aspects of expression of nucleic acid molecules comprising polynucleotide sequences coding for inventive biomarkers described herein.

Alternatively, oligonucleotides or longer fragments derived from nucleic acids encoding each biomarker may be used as targets in a microarray. A number of different array configurations and methods of their production are known to those skilled in the art (see, for example, U.S. Pat. Nos. 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637). Microarray technology allows for the measurement of the steady-state level of large numbers of polynucleotide sequences simultaneously. Microarrays currently in wide use include cDNA arrays and oligonucleotide arrays. Analyses using microarrays are generally based on measurements of the intensity of the signal received from a labeled probe used to detect a cDNA sequence from the sample that hybridizes to a nucleic acid probe immobilized at a known location on the microarray (see, for example, U.S. Pat. Nos. 6,004,755; 6,218,114; 6,218,122; and 6,271,002). Array-based gene expression methods are known in the art and have been described in numerous scientific publications as well as in patents (see, for example, M. Schena et al., Science, 1995, 270: 467-470; M. Schena et al., Proc. Natl. Acad. Sci. USA 1996, 93: 10614-10619; J.J. Chen et al., Genomics, 1998, 51: 313-324; U.S. Pat. Nos. 5,143,854; 5,445,934; 5,807,522; 5,837,832; 6,040,138; 6,045,996; 6,284,460; and 6,607,885).

### Comparing to a control

Once the expression levels of a biomarker or biomarkers of interest (*e.g*., biomarkers selected from Table 1) have been determined (as described above) for a biological sample being analyzed, they can be compared to the expression levels in one or more control samples. Comparison of expression levels according to methods of the present invention is preferably performed after the expression levels obtained have been corrected for both differences in the amount of sample assayed and variability in the quality of the sample used (*e.g*., amount of protein extracted, or amount and quality of mRNA tested). Correction may be carried out using different methods well-known in the art. For example, the protein concentration of a sample may be standardized using photometric or spectrometric methods or gel electrophoresis (as already mentioned above) before the sample is analyzed. In case of samples containing nucleic acid molecules, correction may be carried out by normalizing the levels against reference genes (*e.g*., housekeeping genes) in the same sample. Alternatively or additionally, normalization can be based on the mean or median signal (*e.g.*, Ct in the case of RT-PCR) of all assayed genes or a large subset thereof (global normalization approach).

### Neurodevelopmental Biomarkers

Suitable neurodevelopmental biomarkers may include any developmental biomarker (e.g., cognitive, behavioral or motor skill) that can be used as an indicator of a disease state of Sanfilippo syndrome, the severity of the syndrome, or responses to a therapeutic intervention. Typically, a suitable developmental biomarker has a characteristic that can be objectively measured and evaluated as an indicator. Typically, a suitable developmental biomarker for Sanfilippo syndrome can be used to distinguish between Sanfilippo syndrome patients and normal healthy individuals. Thus, in some embodiments, "differential developmental profiling" may be used to identify developmental biomarkers for Sanfilippo syndrome. As used herein, the term "differential developmental profiling" refers to methods of comparing developmental traits, skills or patterns in two or more subjects (*e.g*., neurodevelopmental data obtained from Sanfilippo syndrome patients vs. control samples obtained from healthy control individuals). Typically, a different developmental trait, skill or pattern observed between the two or more subjects is statistically significant (*i.e.*, the difference is not caused by random variations).

In some embodiments, the developmental biomarker is a neurodevelopmental biomarker. In some embodiments, the neurodevelopmental biomarker is associated with neuromotor function, such as, but not limited to, upper extremity function, lower extremity function, hand eye coordination, neuromuscular response, pain response, temperature response, facial expressions, range of motion and/or overall body movement. In some embodiments, the neruodevelopmental biomarker is associated with neurocognitive function, such as, but not limited to, learning ability, cognitive skill, memory function, reading level, I.Q., short term memory, long term memory, problem solving and/or general analytical ability. In some embodiments, the neruodevelopmental biomarker is associated with speech and language, such as, but not limited to, vocabulary level, pronunciation, annunciation, and/or oral-motor skills. In some embodiments, the neurodevelopmental biomarker is associated with neurobehavioral function, such as, but not limited to, recognition level, attention span and awareness, organization, ability to multitask, activity level and/or self-control. One skilled in the art will appreciate that a large number of standardized tests, surveys or questionnaires may be used to assay any of the neurodevelopmental biomarkers described above. In addition, such standardized tests, surveys or questionnaires may be designed on a case-by-case situation, to address a specific illness, or may be one or more of those currently available within the field. For example, in some embodiments, developmental assessment may be performed using the Bayley Scales of Infant Development III (BSID). In some embodiments, the Kaufman Assessment Battery for Children (KABC) may be used.

In some embodiments, the neurodevelopmental biomarker is associated with neuroanatomical structures and/or their function. In some embodiments, neuroanatomical biomarkers include, but are not limited to, total brain volume, total brain size, brain tissue composition, grey matter volume, white matter volume, cortical volume, cortical thickness, ventricular and CSF volume, cerebella volume, basal ganglia size, basal ganglia volume, frontal lobe volume, parietal lobe volume, occipital lobe volume, and/or temporal lobe volume. In some embodiments, neuroanatomical biomarkers include, but are not limited to, electrical impulse, synaptic firing, neuro-kinetics and/or cerebral blood flow. One skilled in the art will appreciate that a large number of analytical tests may be used to assay any of the structural or functional neuroanatomical biomarkers described above. For example, in some embodiments, neuroanatomical biomarkers may be assayed using X-rays, Positron Emission Tomography (PET), PIB-PET, F18 PET, Single Photon Emission Computed Tomography (SPECT), Magnetic Resonance Imaging (MRI), Functional Magnetic Resonance Imaging (fMRI), Difusion-tensor MRI (DTMRI), Diffusion-weighted MRI (DWMRI), Perfusion-weighted MRI (PWMRI), Diffusion-Perfusion-weighted MRI (DPWMRI), Magnetic Resonance Spectroscopy (MRS), electroencephalography (EEG), magnetoencephalography (MEG), Transcranial magnetic stimulation (TMS), Deep brain stimulation (DBS), Laser Doppler Ultrasound, Optical tomographic imaging, Computer Assisted Tomography (CT) and/or Structural MRI (sMRI). The assay methods described above may be used with or without a contrast reagent, such as a fluorescent or radio labeled compound, antibody, oligonucleotide, protein or metabolite.

As discussed in the Examples section, differential developmental profiling was successfully used to identify developmental biomarkers for Sanfilippo syndrome. In particular, it has been discovered that certain developmental traits and/or phenotypes may be associated with the presence of Sanfilippo syndrome. Accordingly, analysis of developmental biomarkers alone or in conjunction with protein biomarkers can be employed to evaluate risk for Sanfilippo syndrome, detect the presence of Sanfilippo syndrome, monitor progression or abatement of Sanfilippo syndrome, and/or monitor treatment response or optimization.

### Diagnosis of Sanfilippo Syndrome

It is contemplated that one or more biomarkers whose expression profiles correlate with Sanfilippo syndrome can diagnose Sanfilippo syndrome, distinguish between different types of Sanfilippo syndrome (Sanfilippo A, B, C or D), discriminate between different stages of the disease, determine the severity of the disease, and/or assess risk for developing Sanfilippo syndrome. Accordingly, in some embodiments, the present disclosure provides methods for analyzing biological samples obtained from a subject suspected of having Sanfilippo syndrome to measure expression levels of biomarkers described herein, to determine if the subject has Sanfilippo syndrome, is at risk of developing Sanfilippo syndrome, or to determine the severity of the syndrome. Typically, in such methods, the biomarkers' levels determined or measured for a biological sample obtained from the subject are compared to one or more control levels. Various control levels of the biomarkers may be used. For example, suitable control levels may be indicative of the levels of the one or more biomarkers in a control subject who does not have Sanfilippo syndrome. Such control levels may be obtained by measuring the corresponding one or more biomarkers simultaneously under same conditions in a control sample obtained from one or more healthy control subjects. Suitable control samples may be obtained from one healthy control individual or pooled from a plurality of healthy control individuals. In some embodiments, a control level indicative of the level of a biomarker in healthy individuals can be determined from a significant number of individuals, and an average or mean is obtained. Typically, a healthy control individual is at a comparable age or other development state. In some embodiments, a suitable control level for a biomarker is a numerical reference based on historical data, also referred to as a historical control (*i.e.*, of a test or assay performed previously, or an amount or result that is previously known). In some embodiments, a control level is or comprises a printed or otherwise saved record. In some embodiments, an elevated level with statistical significance of the one or more biomarkers as compared to a suitable control level indicates that the subject has Sanfilippo syndrome, is at risk of developing Sanfilippo syndrome. In some embodiments, a diminished level with statistical significance of the one or more biomarkers as compared to a suitable control level indicates that the subject has Sanfilippo syndrome, or is at risk of developing Sanfilippo syndrome. Various statistical techniques and analysis methods may be used to determine if a biomarker has an elevated or diminished level with statistical significance (*i.e.*, the difference is not caused by random variations). Exemplary statistical techniques and methods include, but are not limited to, Linear and Quadradic discriminant analysis, k-nearest neighbor. In some embodiments, a biomarker has an elevated level if the level of the biomarker measured in biological samples is more than 20%, 30%, 40%, 50%, 60%, 70%, 80%, 90%, 1-fold, 1.2-fold, 1.5-fold, 1.75-fold, 2-fold, 2.25-fold, 2.5-fold, 2.75-fold, or 3-fold higher as compared to a control level. In some embodiments, a biomarker has a diminished level if the level of the biomarker measured in biological samples is reduced by more than 10%, 20%, 30%, 40%, 50%, 60%, 70%, 80%, 90%, or 95% as compared to a control level.

In some embodiments, control levels indicative of the levels of the corresponding one or more biomarkers in a control subject who is suffering from Sanfilippo syndrome may be used. Such control levels may be determined by measuring the corresponding one or more biomarkers simultaneously under the same conditions in control samples obtained from a control individual or pooled from a plurality of control individuals. In some embodiments, a control level indicative of the level of a biomarker in individuals suffering from Sanfilippo syndrome can be determined from a significant number of individuals, and an average or mean is obtained. Typically, a suitable control individual is suffering from the same type of Sanfilippo syndrome and at a substantially the same disease and developmental stage (e.g., same age and with similar disease symptoms). In some embodiments, a suitable control level may be a numerical threshold established based on historical data (*i.e.*, of a test or assay performed previously, or an amount or result that is previously known) that correlates with a subject who has Sanfilippo Syndrome, is at risk of developing Sanfilippo syndrome, or is a carrier of Sanfilippo syndrome. In these embodiments, a substantially similar level within statistic error margin or, an elevated or diminished level with statistical significance, of the one or more biomarkers measured in a biological sample as compared to a suitable control level indicates that the subject has Sanfilippo syndrome, or is at risk of developing Sanfilippo syndrome. Various statistical methods and techniques such as those described herein may be used to determine statistical error margin and statistical significance. In some embodiments, a biomarker has an elevated level if the level of the biomarker measured in biological samples is more than 20%, 30%, 40%, 50%, 60%, 70%, 80%, 90%, 1-fold, 1.2-fold, 1.5-fold, 1.75-fold, 2-fold, 2.25-fold, 2.5-fold, 2.75-fold, or 3-fold higher as compared to a control level. In some embodiments, a biomarker has a diminished level if the level of the biomarker measured in biological samples is reduced by more than 10%, 20%, 30%, 40%, 50%, 60%, 70%, 80%, 90%, or 95% as compared to a control level.

In some embodiments, elevated or diminished levels of one or more biomarkers in a biological sample may be used to determine the severity of the syndrome. In some embodiments, the elevated or diminished levels of one or more biomarkers are quantified in order to determine the severity and/or stage of the syndrome.

### Identifying or monitoring treatment

The present disclosure further provides methods for evaluating the effectiveness of a therapy, monitoring responsiveness to therapy, prognosis for disease course, and measurement of disease progression in a subject. Typically, in such methods, levels of suitable biomarkers (e.g., such as those selected from Table 1 and other known markers such as GAG) determined for a biological sample obtained from the subject from one or more time points are compared to the levels from the subject from one or more other time points. For example, biomarker levels may be measured before or at the beginning of a treatment course. Biomarker levels may be measured at one or more time points throughout the course of treatment and compared with the level before the treatment or from an earlier time point of a treatment course. Identification or selection of appropriate treatment, determining if a patient has positive response to a treatment and/or optimization of the treatment can be guided using the information obtained in these methods.

For example, using methods described herein, skilled physicians may select and prescribe treatments adapted to each individual patient based on the diagnosis and disease staging provided to the patient through determination of the expression and/or activity levels of one or more biomarkers described herein (e.g., those selected from Table 1 and other biomarkers for Sanfilippo syndrome such as GAG). In particular, the present disclosure provides physicians with a non-subjective means to diagnose Sanfilippo syndrome early, which will allow for early treatment, when intervention is likely to have its greatest effect, potentially preventing or slowing disease progression and improving patient's quality of life. Selection of an appropriate therapeutic regimen for a given patient may be made based solely on the diagnosis/staging provided by inventive methods described herein. Alternatively or additionally, a physician may also consider other clinical or pathological parameters used in existing methods to diagnose Sanfilippo syndrome and assess its advancement.

In some embodiments, inventive methods described herein can be used for monitoring treatment response in a Sanfilippo syndrome patient. Typically, for example, the levels of one or more biomarkers in a Sanfilippo syndrome patient are measured after receiving treatment for Sanfilippo syndrome. The measured levels are then compared to a control level to determine if the Sanfilippo syndrome patient has positive response to the treatment. As used herein, a "positive response" to a treatment includes reduced severity of disease symptoms, slowed progression, abatement or cure of the disease. A suitable control level may be the level of the one or more biomarkers obtained from the same patient before receiving the treatment or measured at an earlier time point of the treatment. In some embodiments, a suitable control level is the level of the one or more biomarkers in a control patient without the treatment. In some embodiments, such a control level may be determined from a significant number of control patients, and an average or mean is obtained. Typically, a control patient is at a comparable disease or developmental stage. Typically, a diminished or elevated level with statistical significance of the one or more biomarkers as compared to a suitable control level indicates that the patient has positive response to the treatment. Various statistical methods and techniques such as those described herein may be used to determine statistical significance. In some embodiments, a biomarker has a diminished level if the level of the biomarker measured in a biological sample obtained at a relevant time point of interest is reduced by more than 10%, 20%, 30%, 40%, 50%, 60%, 70%, 80%, 90%, 95% as compared to a control level. In some embodiments, a biomarker has an elevated level if the level of the biomarker measured in biological samples is more than 20%, 30%, 40%, 50%, 60%, 70%, 80%, 90%, 1-fold, 1.2-fold, 1.5-fold, 1.75-fold, 2-fold, 2.25-fold, 2.5-fold, 2.75-fold, or 3-fold higher as compared to a control level.

The methods may be applied to all types of Sanfilippo syndrome (e.g., Sanfilippo syndrome Type A, B, C, and D) and various treatment for Sanfilippo syndrome. In particular, inventive methods described herein may be used to identify enzyme replacement therapy as a proper treatment for Sanfilippo syndrome. Using Sanfilippo syndrome Type A as a non-limiting example, a physician may recommend administering a recombinant heparan N-sulfatase (HNS) protein as treatment to a patient based on the level of one or more biomarkers determined using methods described herein. For example, a physician may determine a therapeutically effective amount of the therapeutic agents (e.g., replacement enzymes such as HNS proteins), administration intervals and/or routes, at least in part based on the biomarker levels.

As used herein, the term "therapeutically effective amount" generally refers to an amount that is sufficient to achieve a meaningful benefit to the Sanfilippo syndrome patient, such as an amount sufficient to modulate lysosomal enzyme receptors or their activity to thereby treat such lysosomal storage disease or the symptoms thereof (e.g., a reduction in or elimination of the presence or incidence of "zebra bodies" or cellular vacuolization following the administration of the compositions to a subject). Biomarker levels described herein may be used as a factor for determining a suitable therapeutically effective amount. In some embodiments, biomarker levels may be used in combination with other characteristics of the subject. Such characteristics include the condition, disease severity, general health, age, sex and body weight of the subject.

Biomarker levels described herein may also be used as a factor for determining administration route and/or intervals. In some embodiments, biomarker levels may be used in combination with other factors such as the nature, severity of the disease and extent of the subject's condition. For example, a physician may recommend therapeutic agents (e.g., replacement enzymes such as HNS proteins) be administered intrathecally periodically at regular intervals (e.g., once every year, once every six months, once every five months, once every three months, bimonthly (once every two months), monthly (once every month), biweekly (once every two weeks), weekly) depending on biomarker levels measured with or without considering other factors such as the nature, severity of the disease and extent of the subject's condition. In some embodiments, a physician may also recommend intrathecal administration be used in conjunction with other routes of administration (e.g., intravenous, subcutaneously, intramuscularly, parenterally, transdermally, or transmucosally (*e.g.*, orally or nasally)), depending on biomarker levels measured and/or other factors such as the nature, severity of the disease and extent of the subject's condition.

During the course of various treatment, samples may be obtained from the patient at one or more time points and the levels of suitable biomarkers may be measured and compared to suitable control levels. The doses and frequencies of the treatment may be adjusted to optimize the therapeutic efficacy. Suitable samples for monitoring treatment response may include, but are not limited to, cerebrospinal fluid (CSF), cells, tissue, whole blood (e.g., peripheral blood sample), plasma, serum, blood, and combination thereof. Additional biological samples described above may also be used.

### Kits

The present disclosure further provides kits comprising various reagents and materials useful for carrying out methods according to the present invention. The diagnosis/characterization/staging/monitoring procedures described herein may be performed by diagnostic laboratories, experimental laboratories, or practitioners. The disclosure provides kits which can be used in these different settings.

For example, materials and reagents for characterizing biological samples, measuring biomarker levels (e.g., protein or nucleic acid levels), diagnosing Sanfilippo syndrome in a subject, identifying subtypes, characterizing severity, staging the disease, and/or monitoring treatment response in a subject according to the inventive methods may be assembled together in a kit. In certain embodiments, an inventive kit comprises at least one or more reagents that specifically detects protein or nucleic acid expression levels of one or more biomarkers (*e.g*., those selected from Table 1, other known markers such as GAG, beta-hexosaminidase, LAMP1, LAMP2), and instructions for using the kit according to a method of the invention.

Each kit may preferably comprise the reagent which renders the procedure specific. Thus, for detecting/quantifying a protein marker (or an analog or fragment thereof), the reagent that specifically detects expression levels of the protein may be an antibody that specifically binds to the protein marker (or analog or fragment thereof). For detecting/quantifying a nucleic acid molecule comprising a polynucleotide sequence coding a biomarker, the reagent that specifically detects expression levels may be a nucleic acid probe complementary to the polynucleotide sequence (*e.g*., cDNA or an oligonucleotide). The nucleic acid probe may or may not be immobilized on a substrate surface (*e.g*., beads, a microarray, and the like).

Kits or other articles of manufacture may include one or more containers to hold various reagents. Suitable containers include, for example, bottles, vials, syringes (e.g., pre-filled syringes), ampules. The container may be formed from a variety of materials such as glass or plastic.

In some embodiments, kits may include suitable control levels or control samples for determining control levels as described herein. In some embodiments, kits may include instructions for using the kit according to one or more methods of the invention and may comprise instructions for processing the biological sample obtained from the subject and/or for performing the test, instructions for interpreting the results as well as a notice in the form prescribed by a governmental agency (*e.g*., FDA) regulating the manufacture, use or sale of pharmaceuticals or biological products.

The invention will be more fully understood by reference to the following examples.

### EXAMPLES

### Example 1: Natural History Study and Developmental Assessment of MPSIII A

As discussed above, mucopolysaccharidosis III (MPS-III), also known as Sanfilippo syndrome, is a rare autosomal recessive lysosomal storage disease, caused by a deficiency in one of the enzymes needed to break down the glycosaminoglycan (GAG) heparan sulfate. Heparan sulfate is an important cell surface glycoprotein and a critical component in forming and maintaining the extra-cellular matrix. Four different types of MPS-III (Sanfilippo Syndrome) have been identified: MPS-III A, B, C and D (i.e., Sanfilippo syndrome A, B, C and D). While each of the four MPS-III types display substantially similar clinical symptoms, they are each distinguished by a different enzyme deficiency. MPS-III A (Sanfilippo Syndrome A) has been shown to occur as a result of 70 different possible mutations in the heparan N-sulfatase gene, which reduce enzyme function. MSP-III B (Sanfilippo Syndrome B) has been linked to a disruption in the enzyme N-acetyl-alpha-D-glucosaminidase, MPS-III C (Sanfilippo Syndrome C) to the enzyme acetyl-CoA:alpha-glucosaminide acetyltransferease, and MPS-III D (Sanfilippo Syndrome D) to the enzyme N-acetylglucosamine-G-sulfate sulfatase, all of which are involved in heparan regulation. As a result, each of the enzyme defects causes accumulation of heparan sulfate in Sanfilippo Syndrome patients.

Although pathological cascade is poorly understood, primary accumulation of heparan sulfate triggers secondary accumulation of toxic metabolites, neuroinflammation, disrupted growth factor signaling, dysregulated cell death. Clinical features in Sanfilippo Syndrome patients are overwhelmingly neurological. Typically, a Sanfilippo Syndrome patient has a normal early infancy. Developmental delays often are first manifestations of the disease. Several behavioral disturbances are a prominent feature of mild childhood. Progressive dementia then leads to a "quiet phase" of withdrawal and developmental regression. Typically, a Sanfilippo Syndrome patient survives to late teens or early 20s.

To better understand the pathology underlining Sanfilippo Syndrome, the inventors have conducted a natural history study for MPS-III A. The study was an observational based study with no investigational treatment, to gain insight and develop an understanding of the MPS-IIIA clinical disease spectrum. The second goal of the study was to define a series of clinically definable parameters that could be used to monitor progression of the disease over a 12 month period. Using this approach, the inventors were able to indentify candidate clinical endpoints applicable in clinical trials for future experimental therapy.

For the study, a total of 25 geographically diverse disease subjects (16 males and 9 females), with a confirmed diagnosis of MPS-IIIA were recruited. Each MPS-IIIA subject was required to have a calendar and developmental age, each greater than 1 year. The control group for the study was comprised of 20 young healthy adult subjects, from a wide geographical distribution from across North America. The evaluations were conducted every 6 months over the course of a year. Each MPS-IIIA and control patient, was subjected to a comprehensive neurodevelopmental assessment and brain imaging.

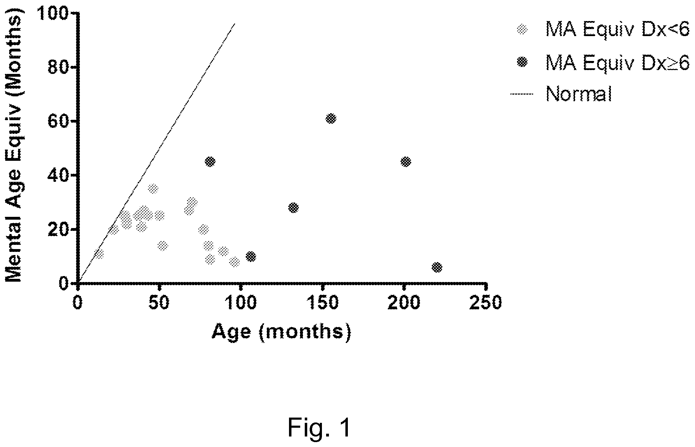

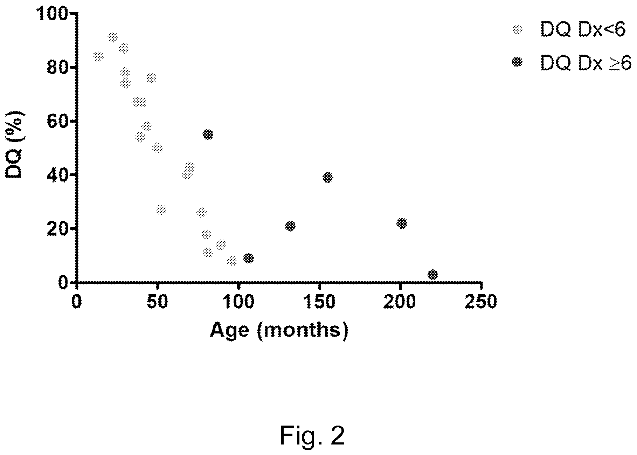

Developmental assessment was performed using either the Bayley Scales of Infant Development III (BSID) or Kaufman Assessment Battery for Children (KABC) approach. For both the BSID and KABC methods, a total mental age equivalent (MA) was calculated in months, for every participant. A subject's developmental quotient (DQ), was determined by dividing a subject's mental age equivalent by their chronological age in months: DQ(%) = (MA/CA)x100. For the study, developmental analysis were carried out on a total of 23 subjects. The first group consisted of 17 subjects, each diagnosed with MPS-IIIA before age 6, with an average DQ of 26 ± 18. The second group consisted of 6 subjects, each diagnosed with MPS-IIIA after age 6, with an average DQ of 52 ± 27. The data was analyzed to compare each subject's calendar age with their experimentally determined mental age (MA) (Figure 1) and developmental quotient (DQ) (Figures 2-3).

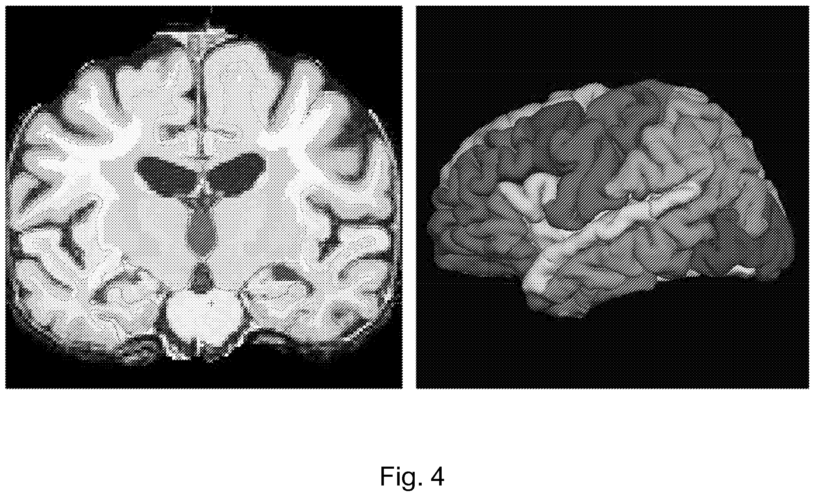

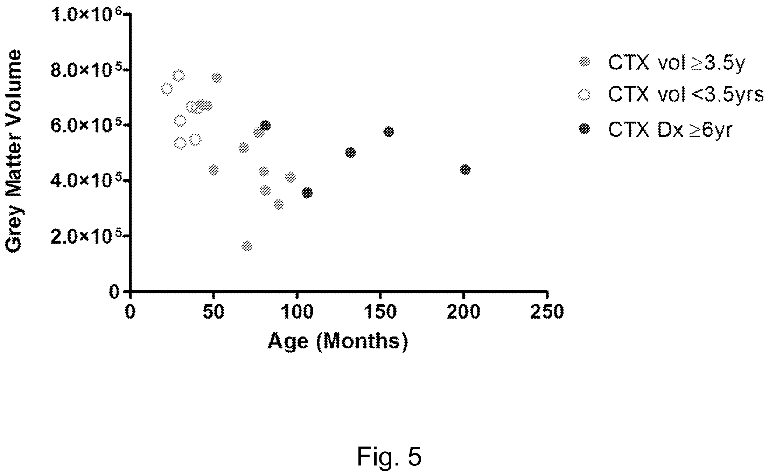

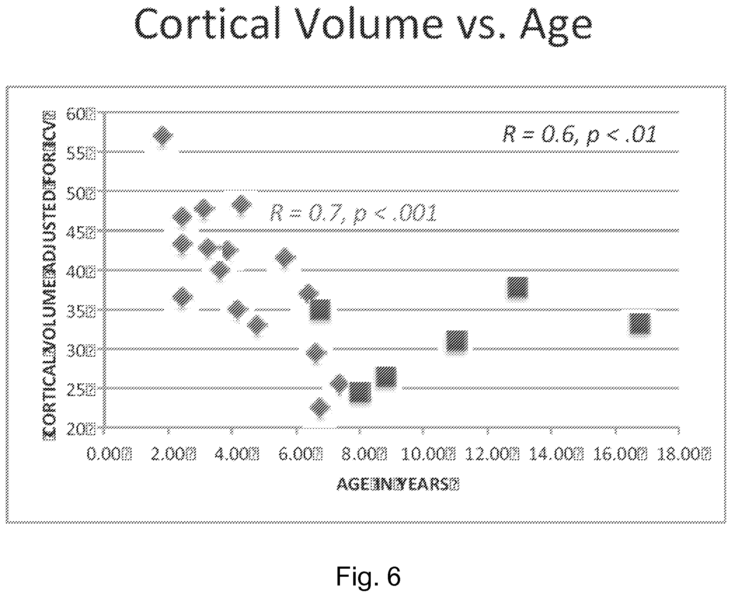

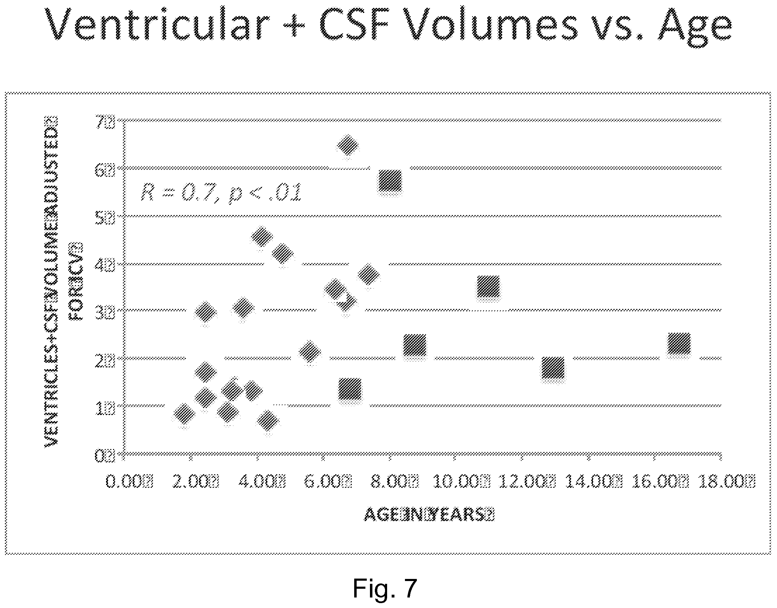

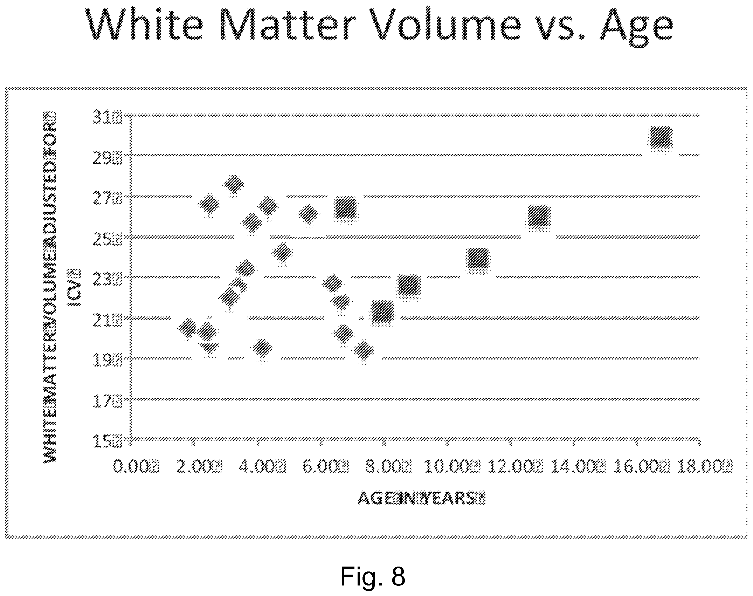

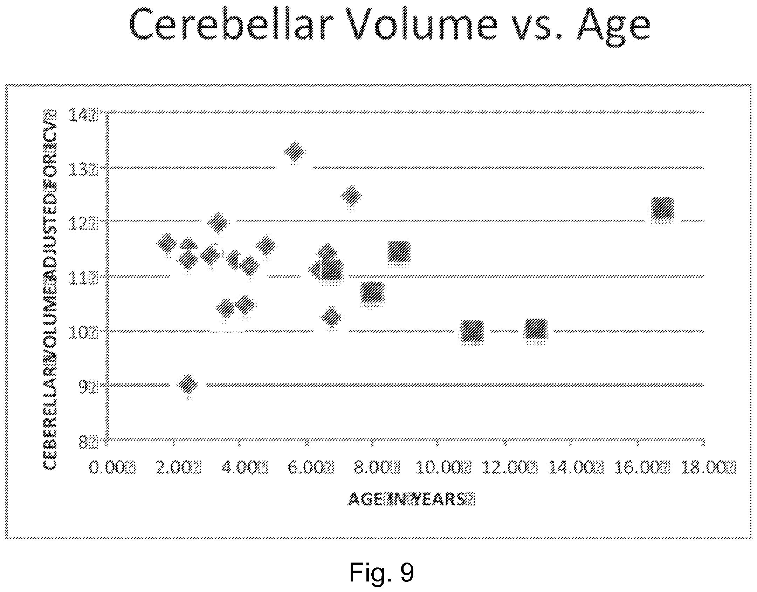

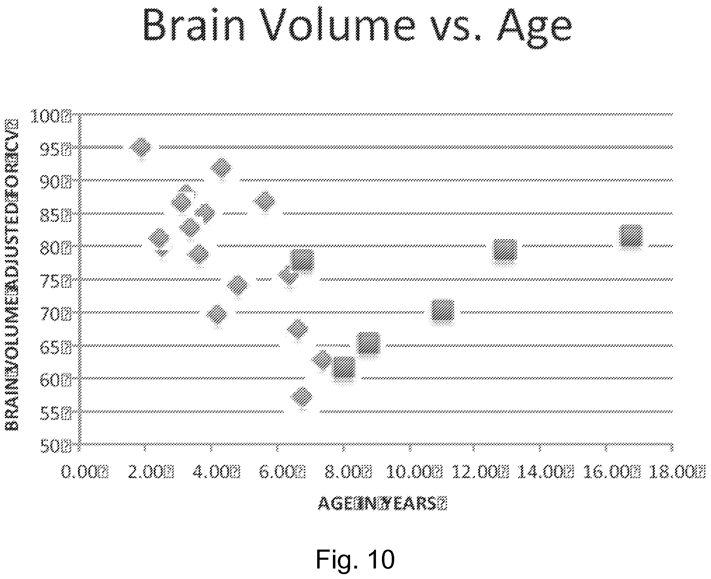

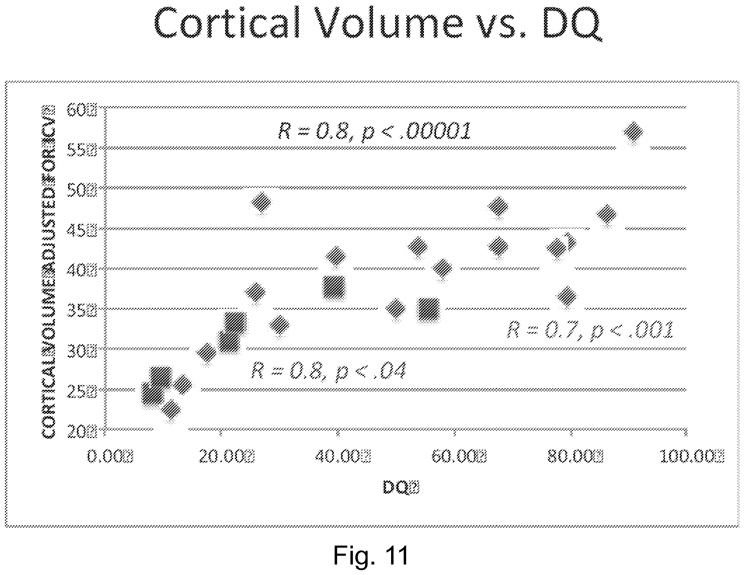

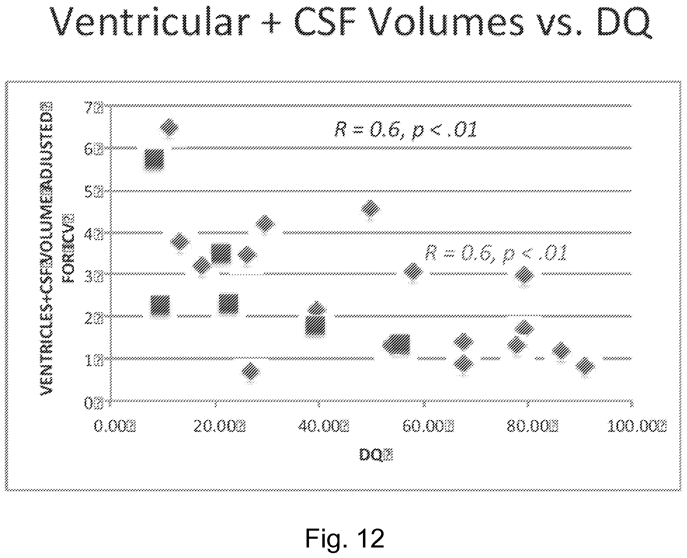

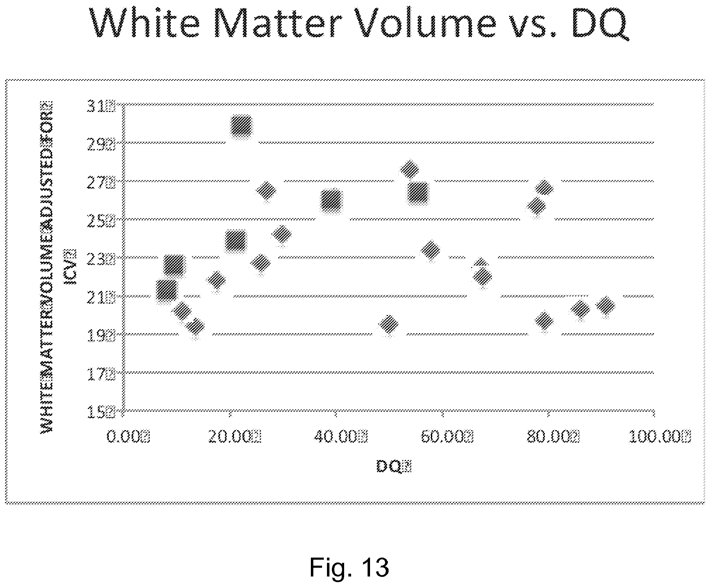

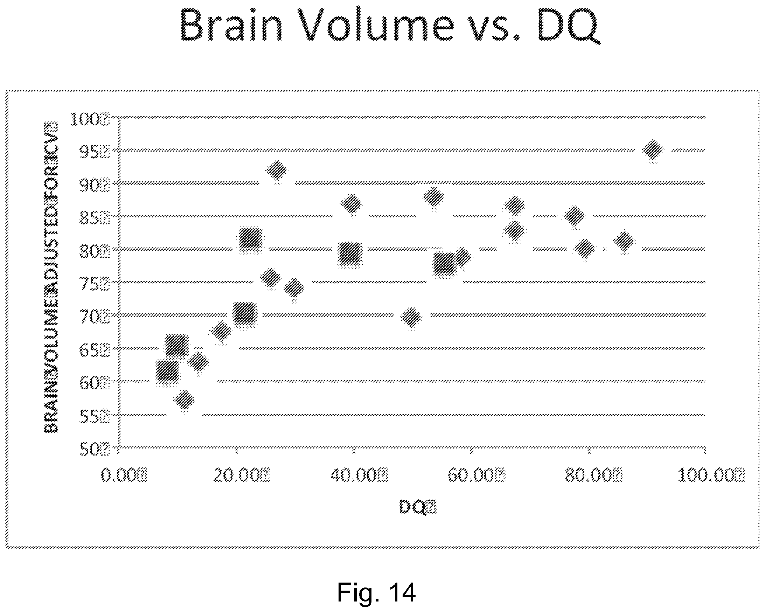

Brain imaging was performed for each subject using non-contrast MRI. For the study, brain scans were carried out on a total of 23 subjects. The first group consisted of 17 subjects, each diagnosed with MPS-IIIA before age 6, with an average age of 4.3 ± 1.7 years. The second group consisted of 6 subjects, each diagnosed with MPS-IIIA after age 6, with an average age of 10.7 ± 3.7 years. Using the images collected, automated volumetric analysis was performed using the FreeSurfer software (Figure 4). Brain volume for the study, was assessed by evaluating several different anatomic criteria such as: Gray matter volume, White matter volume, Cortical volume, Ventricular + CSF volume, Cerebella volume and Total Brain volume). The data was analyzed to evaluate a possible correlation between changes in brain volume over time, when compared to a MPS-IIIA subjects calendar age (Figures 5-10) and development stage (Figures 11-14).

### Observations

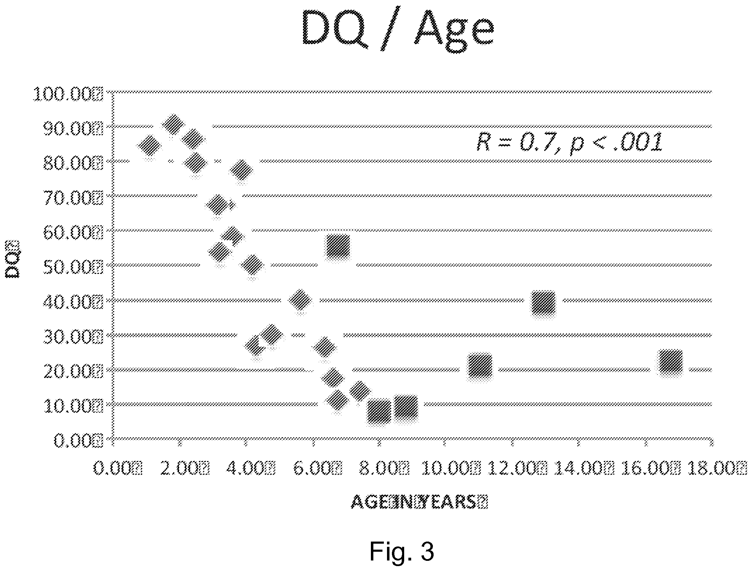

Based on the findings from the study, several key trends were observed. First, a comparison between a subject's baseline developmental stage and age, revealed a possible age-related decline that was different for those MPS-IIIA patients diagnosed before or after age 6 (Figures 1-3). These findings also suggest that those subjects diagnosed with a later onset, may have a slower disease progression. The data also suggests that those patients diagnosed before age 6, display an apparent plateau in mental age at approximately 30 months (Figures 2 and 3). Since children diagnosed before and after the age of 6 years exhibit different patterns of disease progression, it suggest that late diagnosis may be a surrogate for a phenotypic and prognostic difference. This is potentially further supported by the analysis of brain volume, which suggests for those subjects diagnosed with MPSIIIA prior to age 6, there is a potential decrease in cortical volume and an increase in compound ventricular + CSF volumes.

Second, a comparison between brain volume and developmental state, suggests that for those subjects diagnosed with MPSIIIA, there is a decrease in DQ consistent with a reduction in compound ventricular + CSF volume. This reduction was observed in both subjects diagnosed before and after 6 years of age, suggesting the relationship is independent of disease onset. Furthermore, the data also suggest a possible loss in cortical grey matter associated with enlarged ventricles.

### Example 2: Characterization of CSF GAG Levels

The purpose of the study was to determine whether pathological accumulation of heparan suflate glycosominoglycan (GAG) in the brain, is reflective in elevated GAG levels in the cerebrospinal fluid of MPS-IIIA subjects. For the study, a total of 25 geographically diverse disease subjects, with a confirmed diagnosis of MPS-IIIA were recruited. Each MPS-IIIA subject was required to have a calendar and developmental age, each greater than 1 year. The control group for the study was comprised of 20 young healthy adult subjects, from a wide geographical distribution from across North America. The evaluations were conducted every 6 months over the course of a year. Each subject underwent a lumbar puncture to obtain cerebrospinal fluid (CSF) from the lumbar intrathecal space, and the sample was immediately frozen at -80°C in 1 ml aliquots. The biological fluid chosen for the experiment was cerebrospinal fluid, since this medium bathes the central nervous system, and its constituents exist in a dynamic equilibrium with those in the central nervous system.

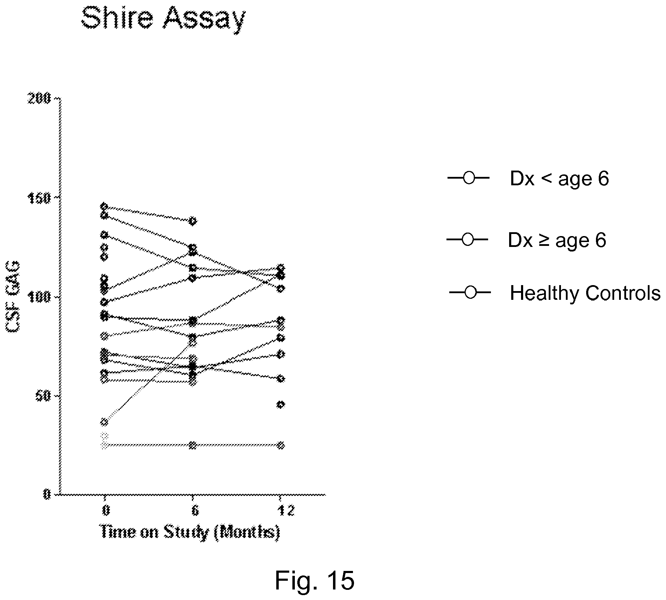

Cerebrospinal glycosaminoglycan levels were measured using standard GAG assay. For example, a GAG assay developed by Applicant is an approach developed to measure a functional attribute of the glycosominoglycan in the sample (Figure 15). The data suggest that the levels of GAGs in the cerebrospinal fluid are significantly increased.

The results from this example indicates that CSF heparan sulfate levels are elevated in Sanfilippo Syndrome patients, and appear to remain at relatively constant levels for up to 12 months. Thus, GAG levels may be used as an indicator to characterize the disease severity and to monitor treatment response.

### Example 3: Identification of biomarkers for Sanfilippo syndrome

### Experimental Design

The inventors have conducted a detailed analysis to identify biomarkers that would reflect the neuropathological consequences of MPS-III in a patient. The biological fluid chosen for the experiment was cerebrospinal fluid, since this medium bathes the central nervous system, and its constituents exist in a dynamic equilibrium with those in the central nervous system. However, one skilled in the art, would appreciate that several other biological fluids can be used for identifying differential biomarkers.

For the study, a total of 25 geographically diverse disease subjects, with a confirmed diagnosis of MPS-IIIA were recruited. Each MPS-IIIA subject was required to have a calendar and developmental age, each greater than 1 year. The control group for the study was comprised of 20 young healthy adult subjects, from a wide geographical distribution from across North America. The evaluations were conducted every 6 months over the course of a year. Each subject underwent a lumbar puncture to obtain cerebrospinal fluid (CSF) from the lumbar intrathecal space, and the sample was immediately frozen at -80°C in 1 ml aliquots. Samples of CSF were analyzed using a Luminex-based multiplexed bead-based immunoassay (Elshal et.al., Methods, 2006, 38:4, 317-323), using fluorescent microbeads containing analyte specific biotinylated detector antibodies. The proteomic evaluation was performed by the service provider Rules-Based Medicine (RBM). Each sample was analyzed to determine the protein concentration level of each of 245 different protein biomarkers from: the RBM Discovery Map 1.0, RBM OncoMap and a custom Neuro/LSD Multiplex. The Neuro/LSD Multiplex comprised the following 13 analyte markers: 14-3-3 protein, three forms of Beta Amyloid, Alpha-Synuclein, Beta-Hexasaminidase, Glial Gibrillary Acidic Protien, Lysosomal-associated membrane protein 1, Lysosomal-associated membrane protein 2, Myelin Basic Protein, Tau, phospho-Tau (p181), and Ubiquitin C-Terminal Hydrolase 1.

### Identification of Protein Differential Biomarkers

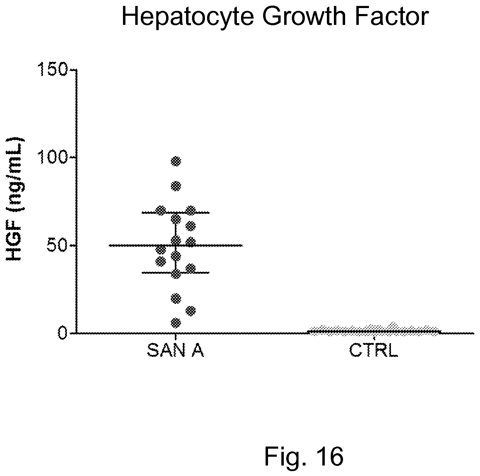

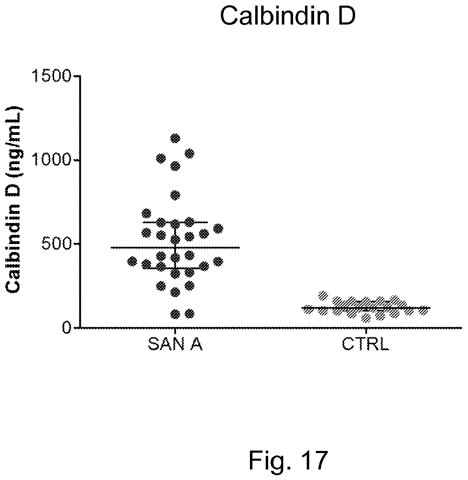

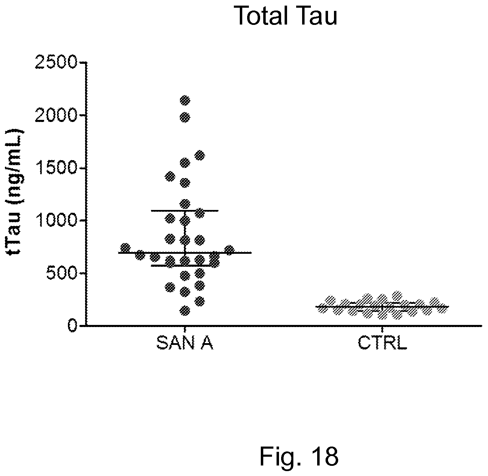

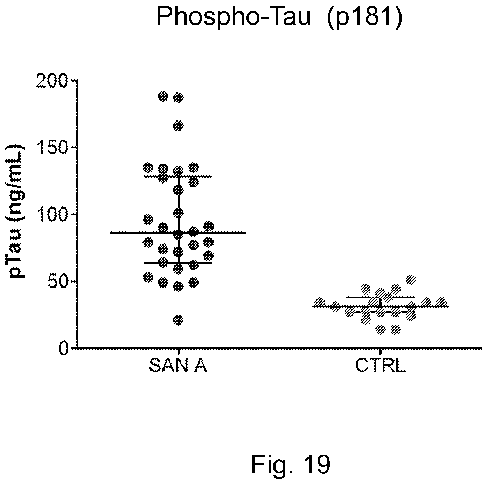

Using the data generated from the bead-based immunoassay, statistical analysis was performed to identify differential protein biomarkers for Sanfalippo Syndrome. For the analysis each of the 245 biomarkers were considered independent of the others using a statistical technique called Discriminant Analysis. In this method, a classification rule is generated from the data, to classify each data point into a group of interest. As such, a desirable classification rule will have a low classification error rate, indicting a desirable biomarker. Under ideal conditions, the classification rule is generated using one dataset and the criterion is tested on an independent dataset. However, since this was not possible, cross-validation error rates were determined by generating and testing the classification rule, by excluding one observation at a time. This produces an almost unbiased estimate of classification error rate. In addition, the data was also analyzed using a Linear and Quadradic discriminant analysis, to exploit a distribution free (or non-parametric) method called k-nearest neighbor, suitable for small sample datasets with high dimensionality. For each of the methods employed, the biomarkers with classification error rates of 10% or less were presented in a side by side fashion. From this approach, 19 biomarkers (Table 1) were identified, which had a classification error rate of ≤ 10% or less by at least one of these methods. Of the 19 identified biomarkers, Hepatocyte Growth Factor, Calbindin D, Total Tau and Phosphorylated Tau (p181), all showed a strong differential expression between the MPS-III and control subjects (Figures 16-19). While the current statistical methods were used for this experiment, one skilled in the art, would appreciate that several other statistical methods could be used for identifying differential biomarkers (see for example, Wu et. al., 2003, Bioinformatics, 19:1636-1643; Biswas et. al., Statistical Advances in Biomedical Sciences; Clinical Trials, Epidemiology, Survival Analysis, and Bioinformatics, 2007; Azuaje, Francisco, Bioinformatics and Biomarker Discovery: "omic" data analysis for personalized medicine, 2010, Jonh Wiley and Sons).

These biomarkers may be used for characterizing Sanfilippo Syndrome, in particular, determining the severity, types and stage of the disease, for monitoring treatment response, and/or evaluating efficacy of a treatment or therapy.

## Claims

1. An *in vitro* method for monitoring the treatment response in a human Sanfilippo syndrome Type A patient, wherein said patient has a constant elevated level of glycosaminoglycan (GAG) in his cerebrospinal fluid (CSF) for up to 12 months prior to treatment as determined relative to the level of GAG in a pooled CSF sample obtained from a plurality of healthy control subjects, said method comprising:
measuring a level of GAG in CSF obtained from the patient after receiving treatment for Sanfilippo syndrome Type A, wherein the treatment comprises administering to the patient recombinant heparin-N-sulfatase (HNS) protein by intrathecal delivery,
wherein the treatment is maintained or adjusted based on the measured level of GAG as compared to the level of GAG measured before or at the beginning of the treatment course for said patient, wherein dose and/or frequency of the treatment is maintained provided that the measured level of GAG is reduced with statistical significance by more than 10% as compared to the control level, indicating that the patient has a positive response to the treatment.

2. The method of claim 1, further comprising measuring a level of one or more neurodevelopmental biomarkers obtained from the Sanfilippo syndrome Type A patient.

3. The method of claim 2, wherein the one or more neurodevelopmental biomarkers comprise a brain anatomical marker, optionally wherein the brain anatomical marker is indicative of brain volume.

4. The method of claim 3, wherein the brain volume is selected from the group consisting of white matter volume, grey matter volume, cortical volume, compound ventricular + CSF volume, cerebella volume, total brain volume and combination thereof.

5. The method of claim 3 or 4, wherein a diminished level of the one or more brain anatomical markers indicates that the patient has a positive response to the treatment.

## Patentansprüche

1. In-vitro-Verfahren zur Überwachung der Behandlungsantwort bei einem menschlichen Patienten mit Sanfilippo-Syndrom Typ A, wobei der Patient für bis zu 12 Monate vor der Behandlung ein konstant erhöhtes Niveau von Glykosaminoglykan (GAG) in seiner Zerebrospinalflüssigkeit (CSF) aufweist, gemäß Bestimmung relativ zu dem Niveau von GAG in einer aus einer Vielzahl gesunder Kontrollprobanden gewonnenen vereinten CSF-Probe, wobei das Verfahren umfasst:
Messen eines Niveaus von GAG in CSF, das aus dem Patienten gewonnen wurde, nachdem dieser eine Behandlung für Sanfilippo-Syndrom Typ A erhielt, wobei die Behandlung die Verabreichung von rekombinantem Heparin-N-Sulfatase(HNS)-Protein an den Patienten durch intrathekale Verabreichung umfasst,
wobei die Behandlung basierend auf dem gemessenen Niveau von GAG verglichen mit dem Niveau von GAG, das vor oder zu Beginn des Behandlungsablaufs für den Patienten gemessen wurde, aufrechterhalten oder angepasst wird, wobei Dosis und/oder Häufigkeit der Behandlung unter der Voraussetzung beibehalten wird, dass das gemessene Niveau von GAG mit statistischer Signifikanz um mehr als 10 % verglichen mit dem Kontrollniveau reduziert ist, was darauf schließen lässt, dass der Patient eine positive Antwort auf die Behandlung zeigt.

2. Verfahren nach Anspruch 1, das weiter das Messen eines Niveaus von einem oder mehreren Biomarkern für die neurologische Entwicklung umfasst, die aus dem Patienten mit Sanfilippo-Syndrom Typ A gewonnen wurden.

3. Verfahren nach Anspruch 2, wobei der eine oder die mehreren Biomarker für die neurologische Entwicklung einen Hirnanatomie-Marker umfassen, wobei wahlweise der Hirnanatomie-Marker auf das Hirnvolumen schließen lässt.

4. Verfahren nach Anspruch 3, wobei das Hirnvolumen aus der Gruppe ausgewählt ist, die aus Weiße-Substanz-Volumen, Graue-Substanz-Volumen, Cortexvolumen, zusammengesetztem Ventrikel+CSF-Volumen, Kleinhirnvolumen, Gesamthirnvolumen und Kombinationen davon besteht.

5. Verfahren nach Anspruch 3 oder 4, wobei ein verringertes Niveau des einen oder der mehreren Hirnanatomie-Marker darauf schließen lässt, dass der Patient eine positive Antwort auf die Behandlung zeigt.

## Revendications

1. Procédé *in vitro* de surveillance de la réponse au traitement chez un patient humain atteint du syndrome de Sanfilippo de type A, dans lequel ledit patient a un taux élevé constant de glycosaminoglycane (GAG) dans son liquide céphalo-rachidien (LCR) pendant jusqu'à 12 mois avant le traitement comme déterminé par rapport au taux de GAG dans un échantillon de LCR groupé obtenu à partir d'une pluralité de sujets témoins sains, ledit procédé comprenant :
la mesure d'un taux de GAG dans du LCR obtenu à partir du patient après avoir reçu un traitement pour le syndrome de Sanfilippo de type A, dans lequel le traitement comprend l'administration au patient de protéine d'héparine-N-sulfatase (HNS) recombinante par administration intrathécale,
dans lequel le traitement est maintenu ou ajusté sur la base du taux mesuré de GAG par rapport au taux de GAG mesuré avant ou au début de la cure de traitement pour ledit patient, dans lequel la dose et/ou la fréquence du traitement est maintenue à condition que le taux mesuré de GAG soit réduit avec signification statistique de plus de 10 % par rapport au taux témoin, ce qui indique que le patient présente une réponse positive au traitement.

2. Procédé de la revendication 1, comprenant en outre la mesure d'un taux d'un ou plusieurs biomarqueurs du neurodéveloppement obtenus à partir du patient atteint du syndrome de Sanfilippo de type A.

3. Procédé de la revendication 2, dans lequel les un ou plusieurs biomarqueurs du neurodéveloppement comprennent un marqueur anatomique cérébral, facultativement dans lequel le marqueur anatomique cérébral est indicatif du volume cérébral.

4. Procédé de la revendication 3, dans lequel le volume cérébral est choisi dans le groupe constitué du volume de matière blanche, du volume de matière grise, du volume cortical, du volume composite ventriculaire + LCR, du volume du cervelet, du volume cérébral total et d'une combinaison de ceux-ci.

5. Procédé de la revendication 3 ou 4, dans lequel un taux réduit des un ou plusieurs marqueurs anatomiques cérébraux indique que le patient présente une réponse positive au traitement.
